# EUROPEAN PATENT SPECIFICATION

(11) **EP 2 676 607 B1**
(45) Date of publication and mention of the grant of the patent: **20.04.2016**
(21) Application number: 12747247.0
(22) Date of filing: 13.02.2012
(51) Int. Cl.: A61B 5/151

(54) **LANCET DEVICE**
LANZETTENVORRICHTUNG
DISPOSITIF DE LANCETTE

(30) Priority: 14.02.2011 JP 2011028944
(43) Date of publication of application: 25.12.2013
(73) Proprietor: Asahi Polyslider Co., Ltd., Osaka-shi, Osaka 530-0005 (JP); Izumi-Cosmo Company, Limited, Osaka-shi Osaka 530-0005 (JP)
(72) Inventor: SAEKI, Hideaki, Maniwa-shi Okayama 719-3226 (JP); SEKI, Kazuharu, Tokyo 108-0074 (JP); ABE, Teruyuki, Tokyo 141-0021 (JP)
(74) Representative: Hoffmann Eitle
(86) International application number: PCT/JP2012/053289
(87) International publication number: WO 2012/111620

(56) References cited:
- WO-A1-2005/102166
- WO-A1-2007/145205
- JP-A- 4 261 645
- JP-A- 2008 523 860
- US-A- 4 924 879
- US-A1- 2003 225 367
- US-A1- 2010 069 943

## Description

### TECHNICAL FIELD

The present invention relates to a pricking device. More specifically, the present invention relates to a lancet device which is used for taking a sample of body fluid such as blood.

### BACKGROUND OF THE INVENTION

In order to measure a blood sugar level of a patient with diabetes, it is required to take a sample of the blood from the patient. The small amount of the taken blood may be enough. Thus, a pricking device capable of taking a small amount of blood is used to measure the blood sugar level. The pricking device is generally composed of a lancet (see, for example, U.S. Patent No. 5,385,571) and an injector. The lancet has a pricking needle capable of puncturing a predetermined region of the patient's body. The injector has a function of launching the lancet toward the predetermined region. The pricking device is set up for use by loading the lancet into the injector. Then, the lancet is launched toward the predetermined region by means of a plunger of the injector, whereby the predetermined region is pricked.

The pricking device used for taking blood from the patient with diabetes is required to be suitable in terms of hygiene and safety. Care should be taken when handling the used lancet. As for the used lancet, there may be the patient's blood adhered to the pricking needle due to the pricking. If the body of a person other than the subject of the blood sampling (for example, a nurse or medical practitioner who collects the blood sample) accidentally should touch the tip of the pricking needle, the body of such person may be pricked by the pricking needle. This will result in a wound of the body through which the patient's blood may enter the body (i.e., the body of the nurse or medical practitioner), and thus posing a risk of the infection disease.

### DISCLOSURE OF THE INVENTION

### PROBLEMS TO BE SOLVED BY THE INVENTION

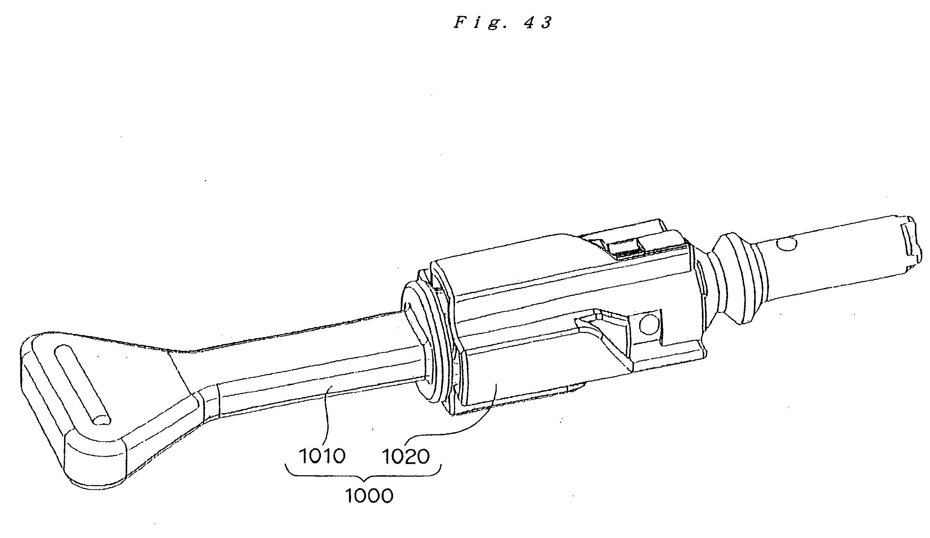

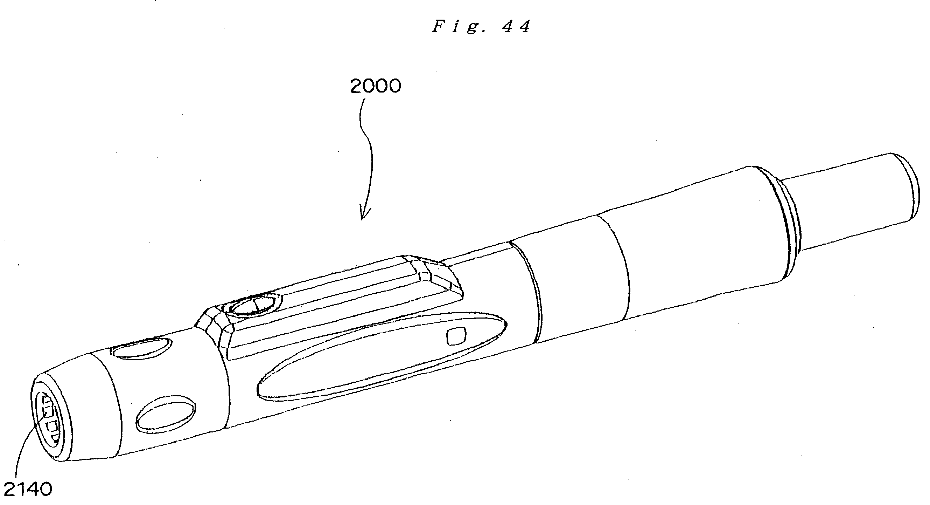

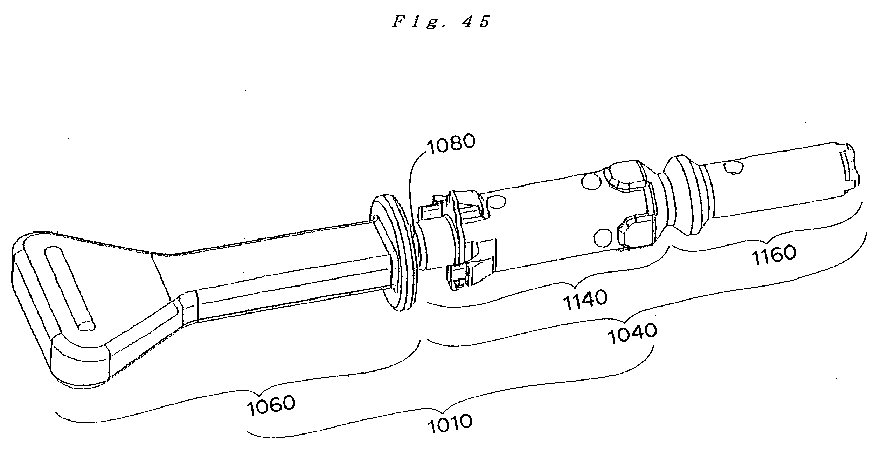

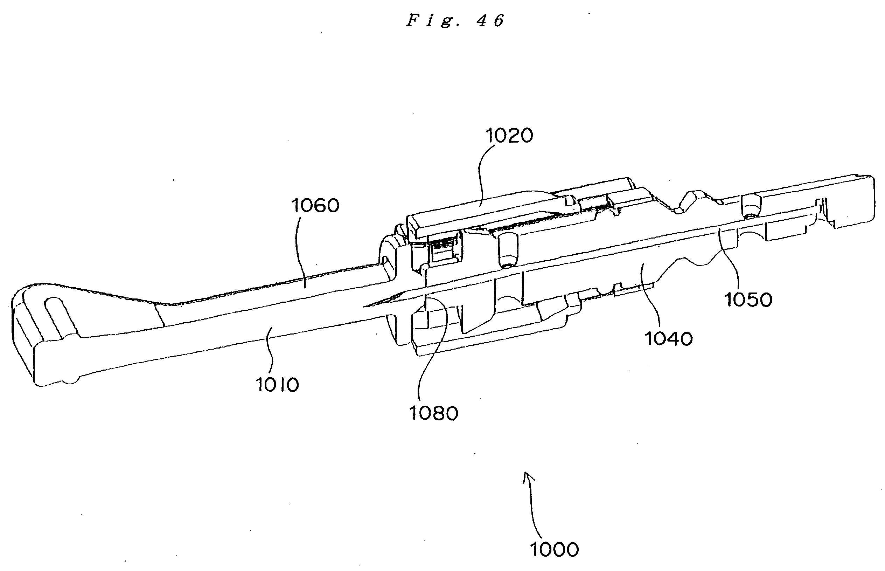

The present applicant has invented the following pricking device, and filed the application regarding such device (WO 2007/018215 A1, filed date: 8 Aug 2006, title of the invention: "PRICKING DEVICE, AS WELL AS LANCET ASSEMBLY AND INJECTOR ASSEMBLY THAT CONSTITUTE THE PRICKING DEVICE"). Referring to the accompanying drawings, the lancet assembly and the injector assembly invented by the applicant will be briefly described below (note that the term "*injector assembly"* will be hereinafter referred to also as "*injector*"). Fig. 43 shows an external appearance of a lancet assembly 1000, and Fig. 44 shows an external appearance of an injector 2000. As shown in Fig. 43, the lancet assembly 1000 is composed of a lancet 1010 and a protective cover 1020. As shown in Figs. 45 and 46, the lancet 1010 comprises a lancet body 1040, a lancet cap 1060 and a pricking needle 1050. The pricking needle 1050 made of metal is disposed in both the lancet body 1040 and the lancet cap 1060 both of which are made of resin. The tip of the pricking needle 1050 is covered with the lancet cap 1060, and the lancet cap 1060 and the lancet body 1040 are integrally connected together by a weakened part 1080. As shown in Figs. 43 and 46, the protective cover 1020 is provided to enclose a part of the lancet body 1040. Such lancet assembly 1000 is loaded into the injector 2000, and then the lancet cap 1060 is removed. By the removal of the lancet cap, the tip of the pricking needle 1050 is exposed, and thereby the lancet can serve to prick.

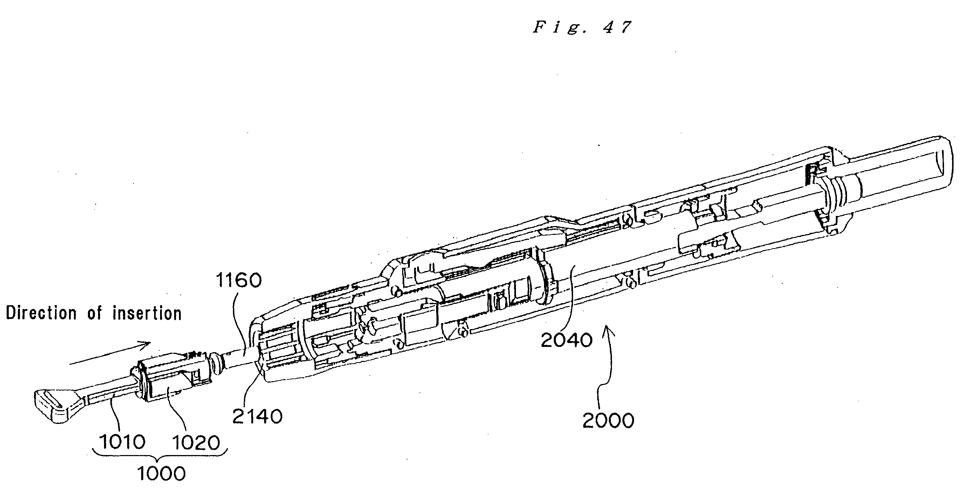

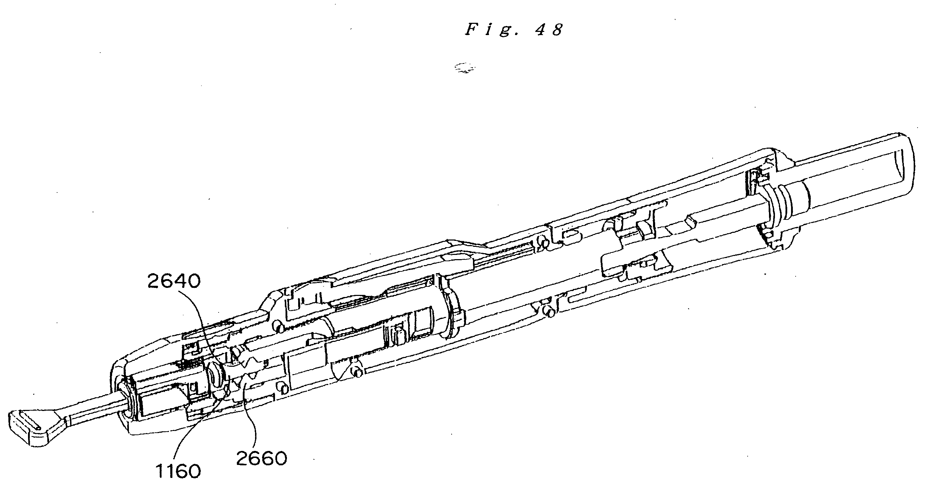

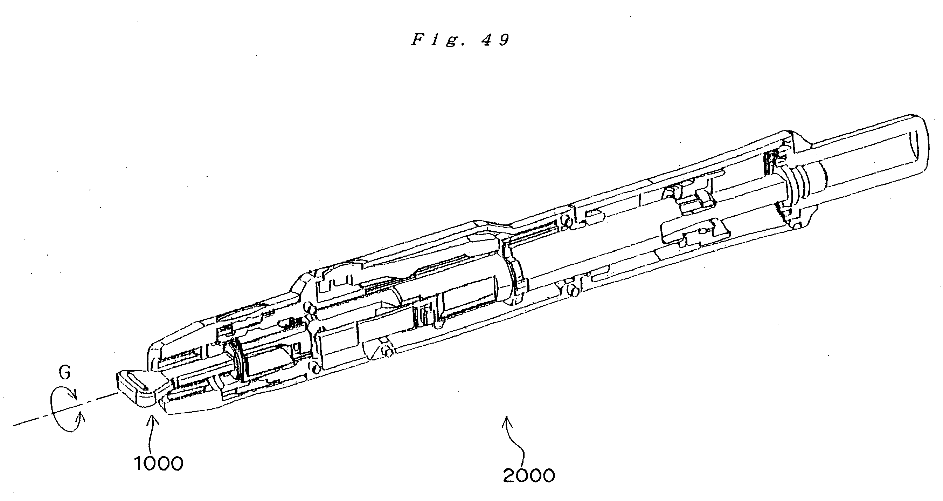

The injector 2000 shown in Fig. 44 can be used in combination with the lancet assembly 1000 to launch the lancet body with the tip of the pricking needle 1050 exposed. The injector 2000 comprises a plunger 2040 that is capable of engaging with a rear end portion of the lancet body to launch the lancet body in the pricking direction (see Fig. 47). As shown in Fig. 47, the lancet assembly 1000 is loaded into the injector 2000 by inserting the lancet assembly 1000 into the injector 2000 through a front end opening 2140 of the injector 2000. As shown in Fig. 48, when the lancet assembly is inserted to some degree, a rear portion 1160 of the lancet assembly 1000 is held by tips 2640 and 2660 of the plunger 2040. Subsequently, when the insertion of the lancet assembly is continued, the plunger 2040 is thrust backward so that the launching energy is stored. That is, the retraction of the plunger 2040 can compress a spring (not shown) provided in the plunger 2040. This means that, when the compression of the spring is released, the plunger instantly moves forward to launch the lancet. Fig. 49 shows the injector 2000 in the state where the plunger has retracted, and the launching energy has been stored therein.

After the loading of the lancet assembly 1000 into the injector 2000 is completed, the lancet cap 1060 is removed to expose the tip of the pricking needle 1050. The removal of the lancet cap 1060 will be described as follows:
As shown in Figs. 45 and 46, the lancet body 1040 and the lancet cap 1060 are integrally connected together by the weakened part 1080 disposed between the lancet body and the lancet cap. The weakened part 1080 is broken by rotating the lancet body 1040 and the lancet cap 1060 around the pricking needle in the reverse direction to each other (see Fig. 49 showing an embodiment of rotating the lancet cap in the direction "G"), whereby the removal of the lancet cap 1060 can be performed.

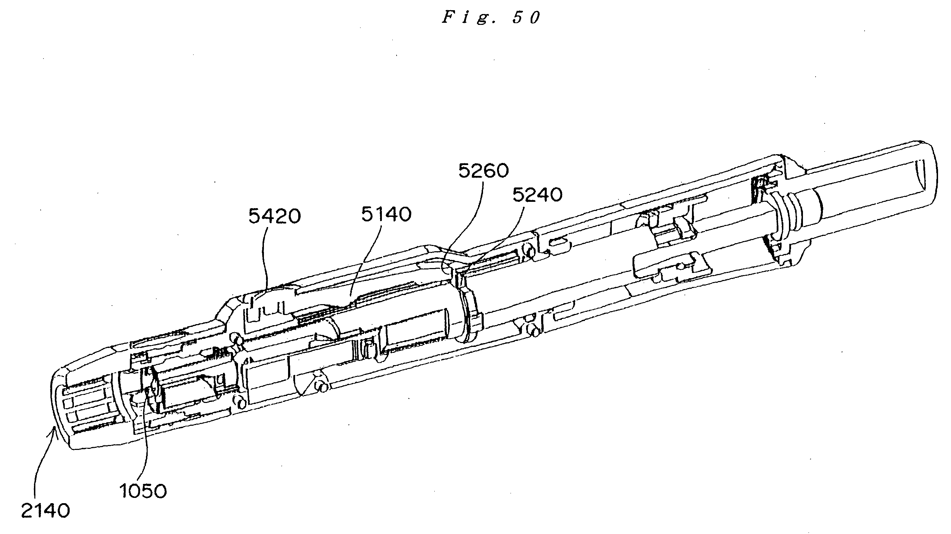

When the pricking operation is carried out, the front end opening 2140 of the injector 2000 is applied to a predetermined region to be pricked (for example, a finger tip). Subsequently, the press part 5420 of a trigger component 5140 is pushed. See Fig. 50. The pushing of the press part 5420 results in an instantaneous expansion of the compressed spring, and thereby forcing the plunger 2040 to move forwardly to prick the predetermined region with the pricking needle.

Upon the pricking operation, it is desired that the pricking needle has a predetermined pathway. In particular, when the pathway of the pricking needle excessively deviates from the linearity, a person from whom blood is to be taken will get worse pain in pricking. Thus, the pathway of the pricking needle is desired to be as linear as possible. Actually, however, the launched pricking needle (especially, a needle tip thereof) is likely to be undulated, which makes it difficult for the pricking needle to surely have the linear pricking pathway. There is also concern that the pricking needle after being used for pricking can be re-used due to the rebound or pulsation of an injection spring. Such re-use of the pricking needle is called "twice pricking phenomenon". The twice pricking phenomenon needs to be addressed to prevent it from occurring.

Furthermore, there is another concern that the used pricking needle can be re-used by getting the retracted state of the plunger 2040 again by some means. That is, if a plunger can be moved backward by some means after the pricking, the pricking device can be again ready for pricking. This means that the lancet after the pricking can be re-used.

The present invention has been devised in view of the above-mentioned circumstances. That is, an object of embodiments of the present invention is to provide a pricking device having an improved pricking pathway of the pricking needle. Another object of embodiments of the present invention is to provide a pricking device which suitably prevents the twice pricking phenomenon regarding the pricking needle. Still another object of embodiments of the present invention is to provide a pricking device having a suitable preventing means for avoiding the re-use of the lancet.

### MEANS FOR SOLVING THE PROBLEMS

According to a first aspect of the present invention, there is provided a lancet device comprising a pricking part and a pressing part, wherein the pricking part comprises a needle shaft component equipped with a pricking needle, a driving shaft component, and an intermediate shaft component therebetween, the needle shaft component, the driving shaft component and the intermediate shaft component being connected to each other via a joint component, wherein the driving shaft component of the pricking part is at least partly rotated by a pressing action of the pressing part, and thereby the intermediate shaft component of the pricking part is displaced causing the needle shaft component to move in a pricking direction, characterized in that the needle shaft component, the driving shaft component, the intermediate shaft component and the joint component therebetween are all in an integrally formed shape with each other in the pricking part, wherein, when the needle shaft component moves in the pricking direction, an elastic portion having a hollow structure of the needle shaft component is brought into contact with an inner wall of a lancet housing so as to receive a force from a surface of a guide on the inner wall of the housing.

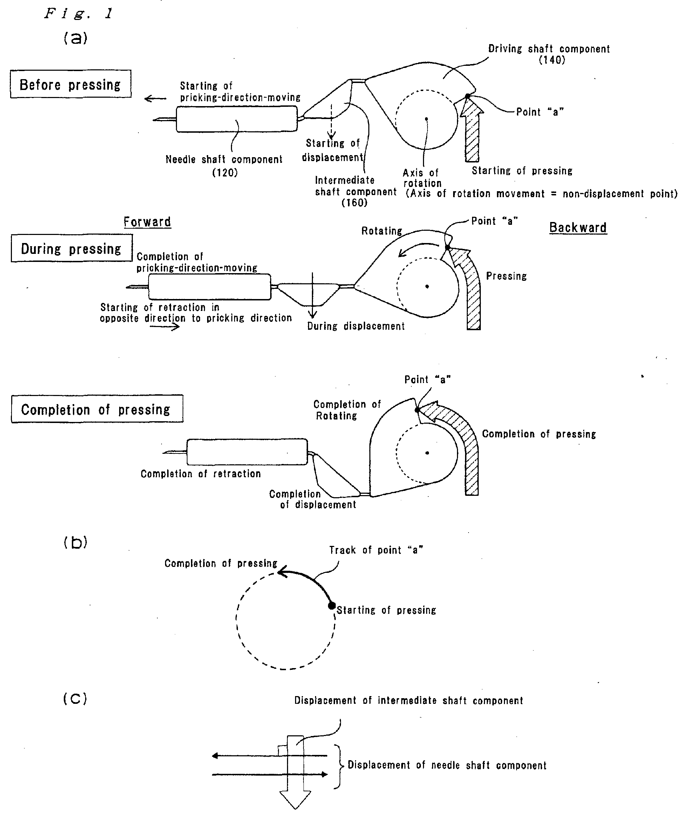

One of features of the lancet device according to embodiments of the present invention is that the pricking part is composed of a plurality of shaft components and a plurality of joint components for jointing the shaft components with each other. In other words, the pricking part has a structure of plural shafts and plural joints. More specifically, the pricking part is comprised of "needle shaft component which is equipped with a pricking needle", "driving shaft component" and "intermediate shaft component provided between the needle and driving shaft components" in the lancet device of the invention. The needle shaft component, the driving shaft component and the intermediate shaft component are coupled to each other via "joint components". The structure of plural shaft components and plural joints in the lancet device of the invention makes it possible that the driving shaft component of the pricking part at least partly rotates by the pressing action of the pressing part to displace the intermediate shaft component of the pricking part, and thereby causing the needle shaft component to move in the pricking direction due to the displacement of the intermediate shaft component. Such movement of the needle shaft component corresponds to a launching of the pricking needle in the pricking direction (see Fig. 1(a)).

The phrase "*at least partly rotated*"/"*at least partly rotate*" as used in the present specification substantially means that, as shown in Fig. 1(a), the rotation of the driving shaft component around an axis thereof is performed not in a full revolution, but in less than the full revolution (for example, less than a half of the full revolution). That is, as shown in Fig. 1(b), a movement track of a pressing point (point "a" shown in the drawing) of the driving shaft component has an arc-like shape.

In particular, the lancet device of the invention has such a function that the needle shaft component of the pricking part moves in the pricking direction and then it retracts in the opposite direction to the pricking direction during the pressing action of the pressing part. That is, both "pricking movement" and "retracting movement after the pricking" of the needle shaft component (i.e., pricking needle) are performed during a unidirectional pressing action of the pressing part.

In a preferred embodiment, a displacement direction of the intermediate shaft component and the pricking direction of the needle shaft component are not in the same line as each other. That is, as shown in Fig. 1(c), the "displacement direction of (movement direction) of the intermediate shaft component" and the "movement directions of the needle shaft component (i.e., pricking direction and the opposite direction thereto)" do not lie in the same straight line. Specifically, as for the displacement of the intermediate shaft component of the pricking part, the intermediate shaft component moves in the direction substantially perpendicular to the pricking direction of the needle shaft component. Preferably, as shown in Fig. 1(a), a rear end of the intermediate shaft component moves in the direction perpendicular to the pricking direction of the needle shaft component such that a front end of the intermediate shaft component serves as an axis of the movement of the rear end.

In a preferred embodiment of the invention, the needle shaft component, the driving shaft component, the intermediate shaft component and the joint components therebetween are all in an integrally formed shape with each other as the pricking part. In this embodiment, it is more preferred that the joint component is flexible. It is also preferred that the joint component is a thin component in the pricking part.

In a preferred embodiment, each of the shaft components of the pricking part has an appropriate form for pricking. For example, a cross-sectional shape of the needle shaft component, taken along a plane parallel to the pricking direction, is rectangular. Namely, the needle shaft component may be generally rectangular in cross section parallel to the pricking direction. A cross-sectional shape of the intermediate shaft component, taken along a plane parallel to the pricking direction, may be trapezoidal. Namely, the intermediate shaft component may be generally trapezoidal in cross section parallel to the pricking direction. Further, a cross-sectional shape of the driving shaft component, taken along a plane parallel to the pricking direction, may have a circular shape in at least portion of the shape.

In a preferred embodiment, the pressing part is a spring part. In this embodiment, during the spring part in a compressed state is being released to extend (i.e., while the compressed spring returns to its original state due to the releasing of the compressed state), the driving shaft component is pressed in a rotation direction thereof by the pressing part, and thereby the intermediate shaft component of the pricking part is displaced. Such displacement of the intermediate shaft component causes the needle shaft of the pricking part to move both in the pricking direction and an opposite direction thereto.

In a preferred embodiment, the lancet device further comprises a housing for accommodating the pricking part and the pressing part therein. It is preferred in this embodiment that the housing has, on its inner wall, a guide rail for a slide movement of the needle shaft component. The needle shaft component preferably moves in the pricking direction and the opposite direction thereto, sliding on the guide rail of the housing. Preferably, the guide rail is composed of two opposed elongate portions which protrude vertically with respect to the inner wall of the housing. The needle shaft component is provided with an elastic portion having a hollow structure. In this case, it is preferred that, when the needle shaft component moves in the pricking direction and the opposite direction thereto, the elastic portion of the needle shaft component slides on the guide rail of the housing.

In a preferred embodiment, the lancet device further comprises a trigger part. It is preferred in this embodiment that a rear end of the trigger part is in abutment (contact) with the driving shaft component of the pricking part such that the spring part coupled to the driving shaft component is held in its compressed state (at a point in time before a pricking operation). When a front portion of the trigger part is pushed toward an inside of the housing in order to perform the pricking operation, a ceasing of the abutment (contact) of the rear end of the trigger part with the driving shaft component of the pricking part is preferably caused, and thereby the driving shaft component is preferably pressed in the rotation direction thereof by the spring part.

It is preferred in the lancet device that the pricking part is equipped with a needle cap for protecting the tip of the pricking needle, and the trigger part is equipped with a movement-inhibiting component for inhibiting an movement of the pricking part (at a point in time before a pricking operation). It is preferred that the movement-inhibiting component of the trigger part and the needle shaft component of the pricking part are capable of making abutment (contact) with each other, and thereby a displacement (or movement) of the needle shaft component is prevented during a removal of the needle cap from the tip of the pricking needle.

The lancet device of the invention preferably has a thin compact shape as a whole. For example, the thickness dimension of the lance device according to the present invention is for example in the approximate range of 3 to 15 mm, and more preferably in the approximate range of 4 to 9 mm (for example, about 6 mm).

According to one embodiment of the present invention, the lancet device further comprises a case-shaped trigger part and a housing. The pricking part is accommodated within the case-shaped trigger part. The case-shaped trigger part in which the pricking part is accommodated is surrounded by the housing such that only a tip portion (front end portion) of the trigger part is exposed from the housing. In this embodiment, the case-shaped trigger part comprises a first flexible portion which is in abutment (contact) with the driving shaft component of the pricking part at a point in time before a pricking operation, such that the spring part coupled to the driving shaft component is held in its compressed state. When the tip portion of the case-shaped trigger part is pushed toward an inside of the housing in order to perform the pricking operation, the first flexible portion of the case-shaped trigger part is forced to slide on a first continuous projecting portion provided on an inner wall of the housing. Due to such sliding, the first flexible portion is displaced outwardly to cease the abutment of the first flexible portion with the driving shaft component. As a result, the driving shaft component is pressed in the rotation direction thereof by the spring part. As for the embodiment wherein the lancet device further comprises the case-shaped trigger part and the housing, the tip portion of the case-shaped trigger part cannot be pushed toward the inside of the housing due to an abutment between the needle cap and the housing at a point in time before the needle cap is removed from the pricking part, and whereas the tip portion of the case-shaped trigger part can be pushed toward the inside of the housing at a point in time after the needle cap is removed from the pricking part. The case-shaped trigger part may further comprise a second flexible portion in addition to the first flexible portion. In this case, the second flexible portion is in engagement with the needle shaft component at a point in time before the pricking operation, and thereby a displacement of the needle shaft component is prevented during a removal of the needle cap from the tip of the pricking needle. When the tip portion of the case-shaped trigger part is pushed toward the inside of the housing in order to perform the pricking operation, the second flexible portion of the case-shaped trigger part is forced to slide on a second continuous projecting portion provided on the inner wall of the housing. Due to such sliding, the second flexible portion is displaced outwardly to cease the abutment of the second flexible portion with the needle shaft component. The case-shaped trigger part preferably has, on its inner wall, a guide rail for a slide movement of the needle shaft component. Thus, when the needle shaft component moves in the pricking direction and the opposite direction thereto, the needle shaft component can slide on the guide rail of the case-shaped trigger part.

### EFFECTS OF THE INVENTION

### (Linearity of Pricking Needle)

The lancet device has a straight pathway of the pricking needle during the pricking action. That is, the pricking needle moves straightly in the pricking direction and then straightly retracts without wobbling. As a result, the pain of the pricked subject (i.e., a person from whom blood is taken) can be largely relieved upon pricking. This means that the pain felt by the person to be pricked (i.e., the subject of the blood sampling) can be largely reduced at the time of the pricking. While not wishing to be bound by any theory, the reduced pain is believed to be attributed to a reduction of "adverse hollowing or scratching phenomenon of the pricked portion by the wobbling needle".

In particular, the lancet device has a large force for moving the needle shaft component in the pricking direction. The reason for this is that the moving of needle shaft component in the pricking direction is caused by the intermediate shaft component displaced due to the rotation of the driving shaft component. Because of such large force, even while the needle shaft component is in strong abutment against the guide rails, the needle shaft component can slide in the pricking direction, which appropriately ensures the linearity of the needle shaft component. That is, the needle shaft component of the lancet device can forcefully move in the pricking direction, even when the needle shaft component is largely pressed against the guide rail, causing a large sliding resistance between the needle shaft component and the guide rails. This means that the lancet device appropriately achieves the linearity of the needle shaft component, the linearity being based on the guide rails.

In this regard, the conventional lancet device (i.e., lancet device in which a plunger presses a pricking needle to force it to move in a pricking direction) does not have a large force for moving the pricking needle in the pricking direction. Thus, the movement of the pricking needle might be prevented on the way by a large sliding resistance between the needle and a guide. The exposed state of the pricking needle, which is exposed from a pricking opening, might also be adversely held by the large sliding resistance. In order to avoid such inconveniences, it is required in the conventional lancet device that oil is applied to a sliding surface. It is also required that parts with high accuracy of dimension are used. In these regards, the following means are proposed to directly avoid the sliding resistance.
- A force for pressing the pricking needle against the guide is decreased; and
- A slight space is provided between the pricking needle and the guide.

Those means, however, might diminish the linearity based on the guide, causing the wobble of the pricking needle.

In contrast to such conventional lancet device, embodiments of the invention makes it possible to smoothly move the needle shaft component in the pricking direction because of a large moving force of the needle shaft component even while the needle shaft component is strongly being pressed against the guide rail, which sufficiently exhibits the linearity based on the guide rail. From another viewpoint, the lancet device according to embodiments of the invention includes the integrally formed components. That is, the needle shaft component, the driving shaft component, the intermediate shaft component, and the joint components therebetween are integrally formed together. As a result, the driving force caused by the pressing part is effectively transferred to the needle shaft component, and thus the needle shaft component can have the large moving force in the pricking direction. Embodiments of the invention appropriately makes use of the large moving force of the needle shaft component to achieve the linearity of the needle.

### (Prevention of Needle Wobble at the Time of Pricking)

According to embodiments of the present invention, the needle shaft component equipped with the pricking needle moves in the pricking direction and then it moves in the opposite direction to the pricking direction during a pressing action of the pressing part. These movements are a smooth sequence of moving steps. That is, the exposure of the pricking needle from the pricking opening, and the retracting of the pricking needle are smoothly performed. This means that the movement of the pricking needle is smooth at the time of pricking. The linearity of the pricking needle, which is described above, is appropriately kept in pricking. In this regard, the conventional lancet device restricts the exposed length of the pricking needle from the pricking opening by allowing the pricking needle to collide with the edge around the pricking opening of the device (for example, an inner wall of the housing), which might cause the wobbling of the pricking needle due to the collision. In contrast, embodiments of the invention can appropriately avoid such "needle wobble" upon pricking since the adjustment of the exposed length of the pricking needle is not performed by such "collision of the pricking needle".

Due to no "collision of the pricking needle", the lancet device of the embodiments of the invention can suppress the "wobble" of the pricking needle, which leads to an achievement of the stable pricking pathway. According to embodiments of the present invention, the pricking pathway of the needle becomes substantially constant even when being used by a different user. There is accordingly provided such an advantageous effect that the fluctuations of the pricking pathway, attributed to the different users, can be effectively reduced.

### (Prevention of Twice Pricking Phenomenon)

Embodiments of the invention perform both "exposure of the pricking needle from the pricking opening" and "retraction of the pricking needle" while the compressed spring is being released to extend until it has its original state. That is, the lancet device does not employ the conventional mechanism. In the conventional device, the pricking needle is exposed due to the extension force of the compressed spring and the pricking needle is retracted by the reverse force with which the extended spring returns to its original state. In this regard, embodiments of the invention can appropriately prevent the "twice pricking phenomenon" of the pricking needle. More specifically, in the conventional device, the pricking needle after being exposed might be exposed again from the pricking opening due to the spring pulsation caused after the releasing of the compressed spring. In contrast, embodiments of the present invention allow the needle shaft component equipped with the pricking needle to be deeply retracted into the device at the time of the spring pulsation, if any. And also, a weak force of the spring pulsation, which may occur in the device, cannot permit the intermediate shaft component to be displaced backwardly in the direction opposite to the direction for pricking.

### (Prevention of Re-use)

According to embodiments of the invention, the intermediate shaft component is unidirectionally displaced to achieve both the exposure of the pricking needle from the pricking opening and the retraction of the pricking needle while the compressed spring is released to extend until the spring has its original shape. In order to return the used pricking part and spring, which have been already used for pricking, to their original state (i.e., their state provided at a point in time before the pricking operation), it is necessary to disassemble the device as a whole. This means that the lancet device has such a structure that prevents the re-use of the pricking needle after being used. Accordingly, the lancet device makes it impossible to re-use the pricking needle after being used, which is desirable from the viewpoint of hygiene and safety. The user has no choice but to use the lancet device as "non-reusable type device" or "disposable type device", which can inevitably ensure the hygiene of the person from whom blood is taken.

### (Preferable Size)

The lancet device may be substantially composed of four parts, namely, the "pricking part", the "pressing part", the "housing", and the "trigger part". Thus, the device structure is very simple and also has the small size as a whole. Specifically, the lancet device has the relatively simple structure mainly including the "lancet pricking part" and the "spring" accommodated in the lancet housing, and the pricking part has a small thickness of about 1.5 to 4.5 mm (for example, about 3 mm) that determines the thickness of the entire device. As a result, the device is easy to manufacture, and also is superior in carrying efficiency and storage space. Further, the device has such a size that can be held with the fingers, which is preferable from the viewpoint of operability when the device is actually used.

### BRIEF DESCRIPTION OF THE DRAWINGS

In order to better understand the present invention, and to show how the same may be carried into effect, reference will now be made, by way of example only, to the following drawings.
Fig. 1 is a schematic diagram showing the concept of a lancet device according to embodiments of the present invention.

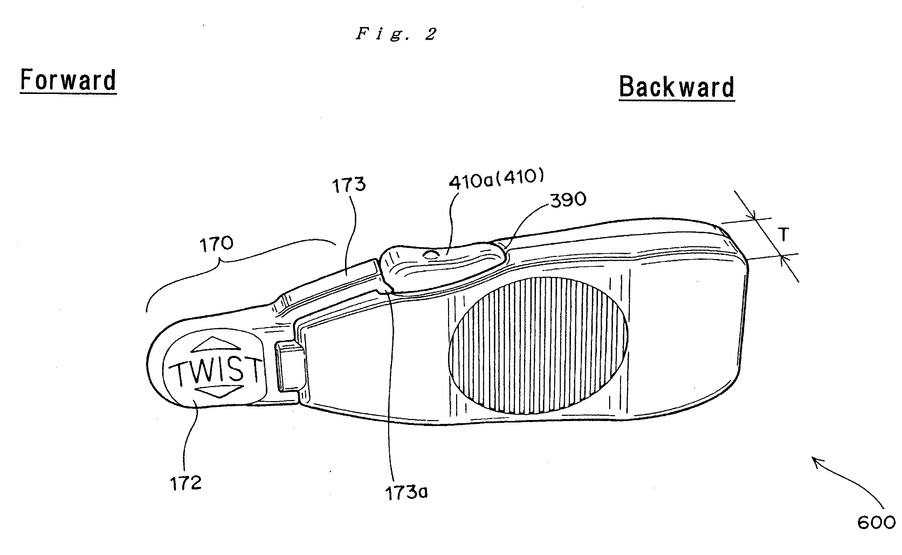

Fig. 2 is a perspective view showing an appearance of a lancet device 600 according to embodiments of the present invention.

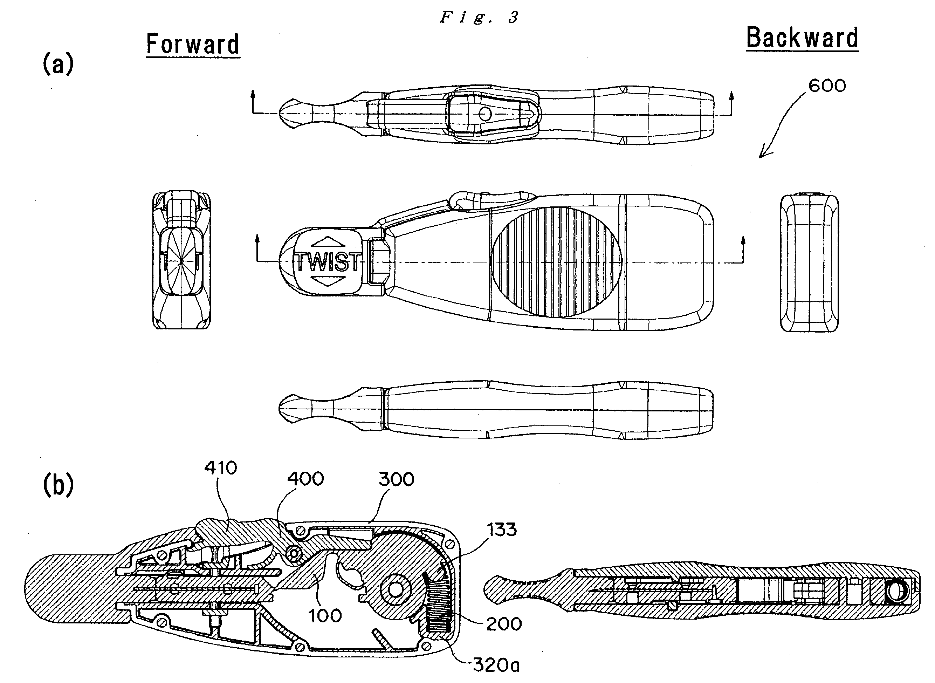

Fig. 3 shows an appearance view, a side view and a cross-sectional view of the lancet device 600 according to embodiments of the present invention.

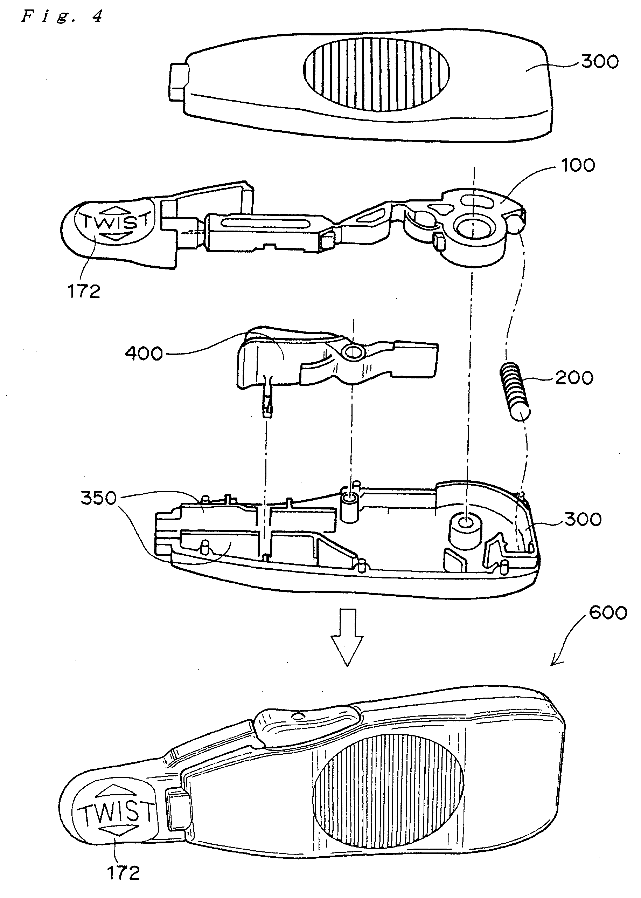

Fig. 4 shows an exploded perspective view and an appearance perspective view of the lancet device 600 according to embodiments of the present invention.

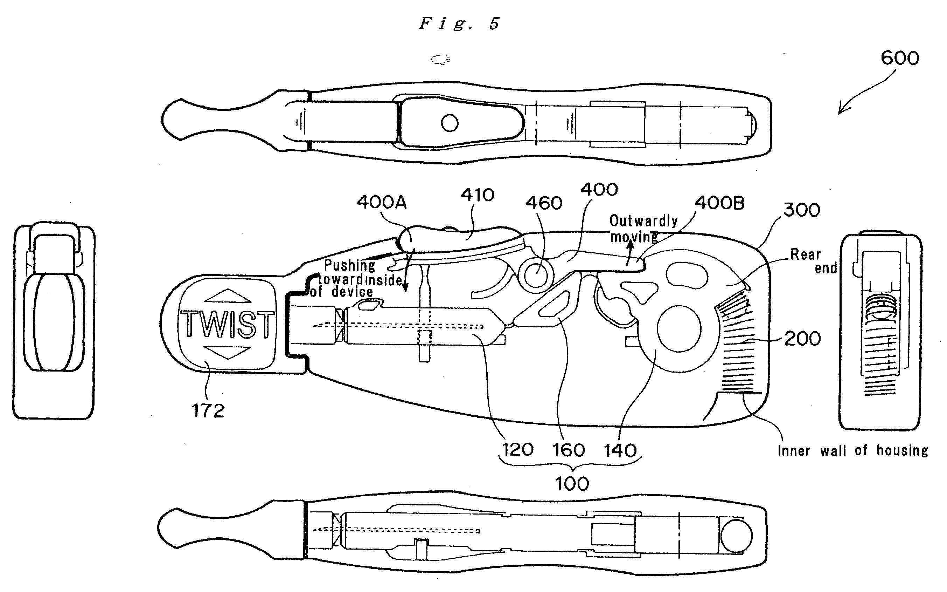

Fig. 5 is a transparent view showing an internal structure of the lancet device 600 according to embodiments of the present invention.

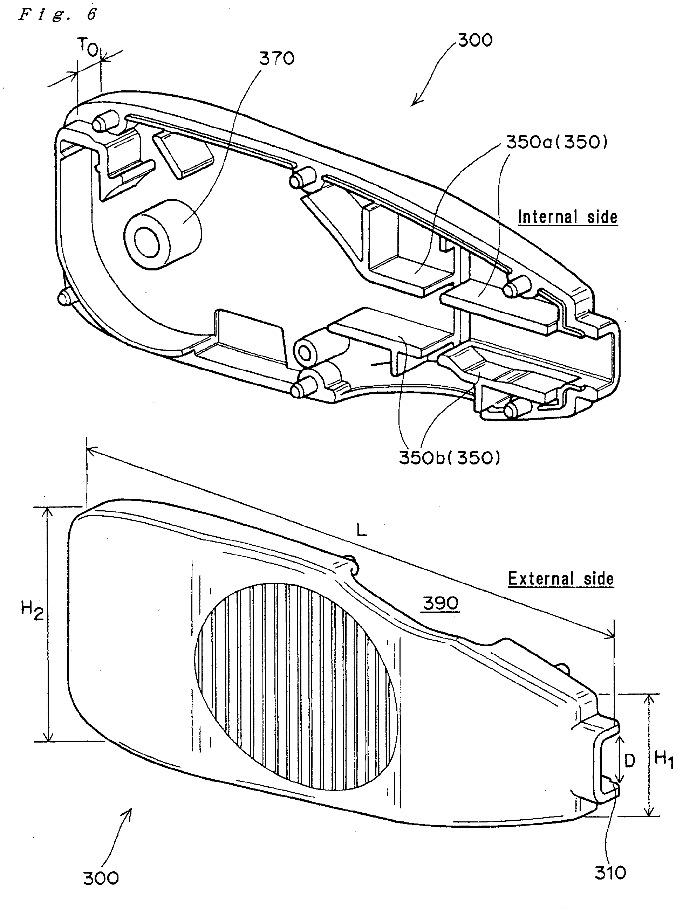

Fig. 6 is a perspective view of a housing (one of half parts of the housing) used for the lancet device 600.

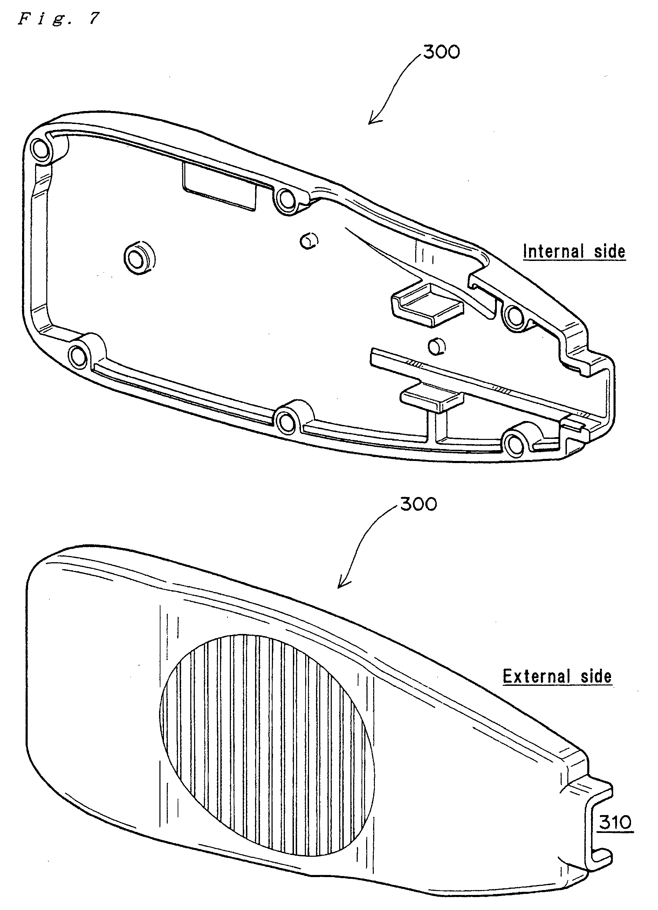

Fig. 7 is a perspective view of the housing (the other of the half parts of the housing) used for the lancet device 600.

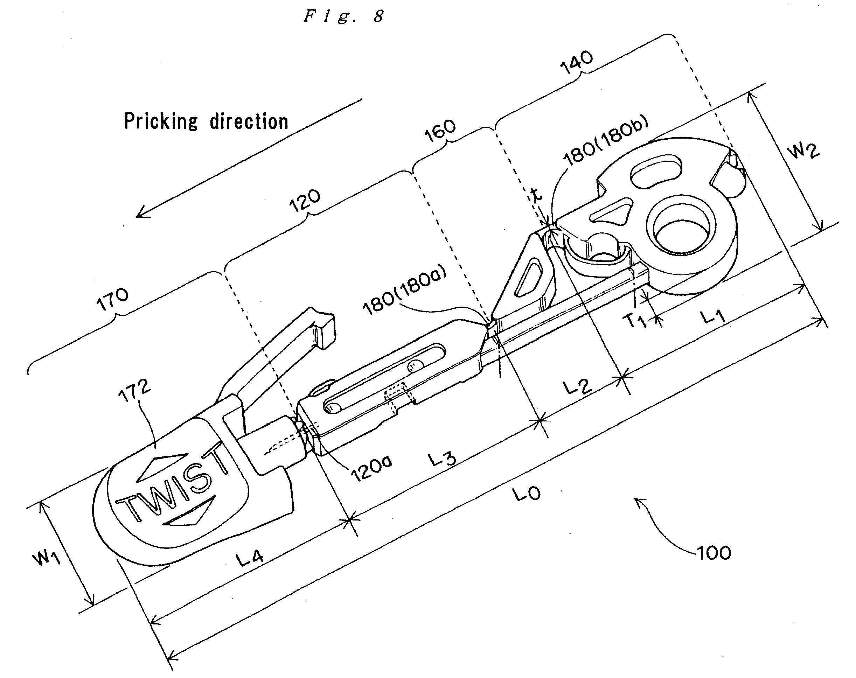

Fig. 8 is a perspective view of a pricking part (lancet pricking part) used in the lancet device 600.

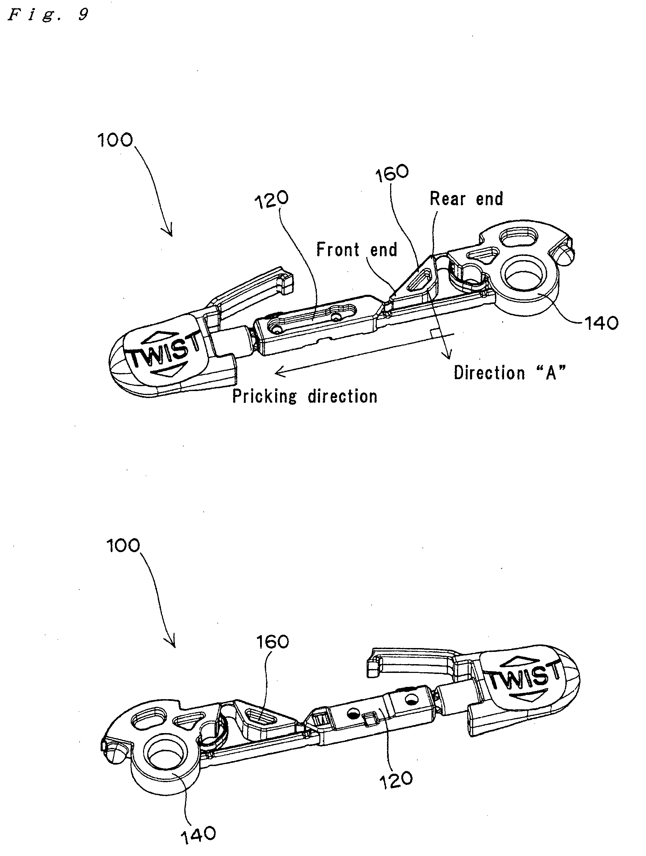

Fig. 9 is a perspective view of the pricking part (lancet pricking part) used in the lancet device 600.

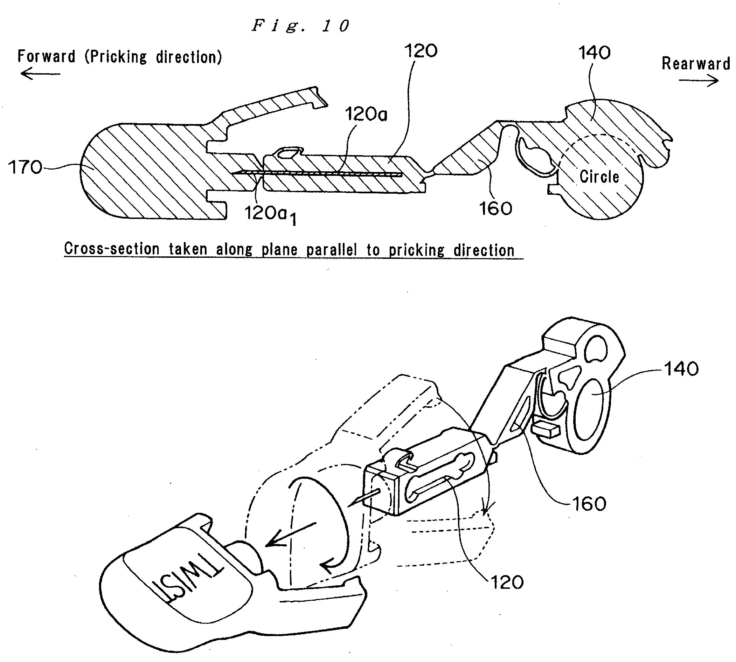

Fig. 10 shows a cross-sectional view and a perspective view of the pricking part (lancet pricking part) used in the lancet device 600.

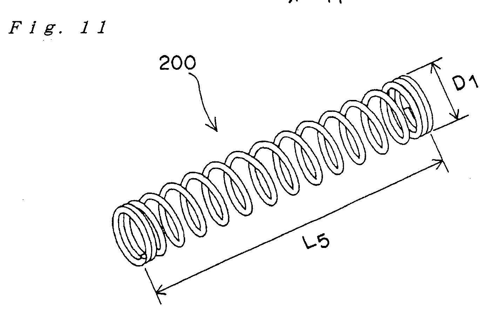

Fig. 11 is a perspective view of a pressing part (e.g., spring) used in the lancet device 600.

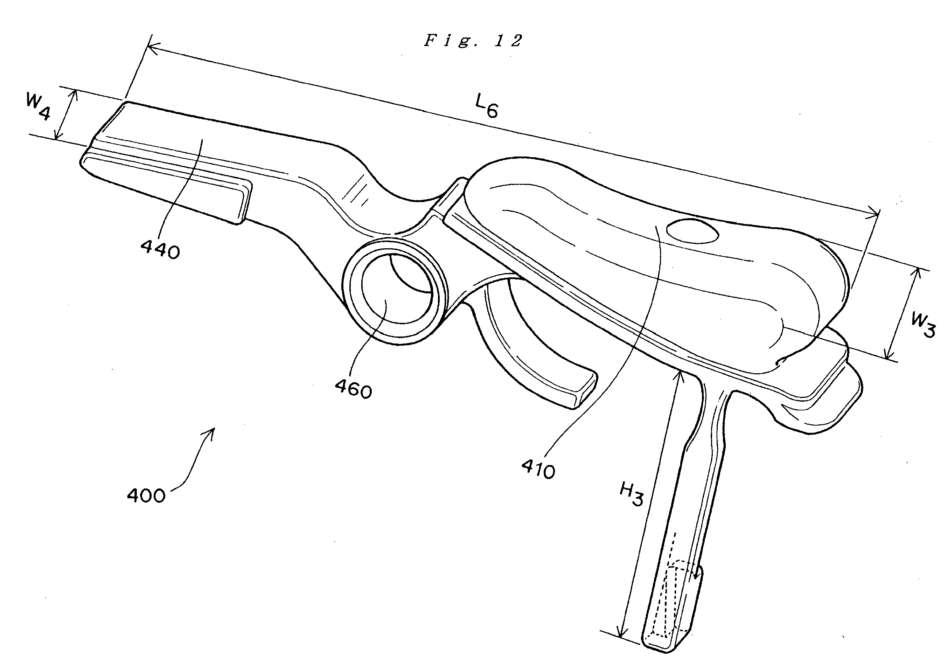

Fig. 12 is a perspective view of a trigger part used in the lancet device 600.

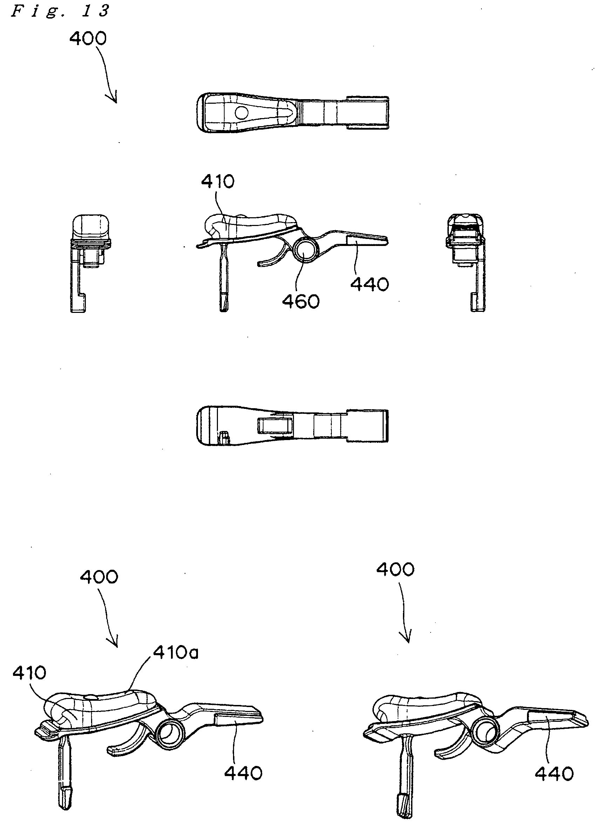

Fig. 13 shows a perspective view and a side view of the trigger part used in the lancet device 600.

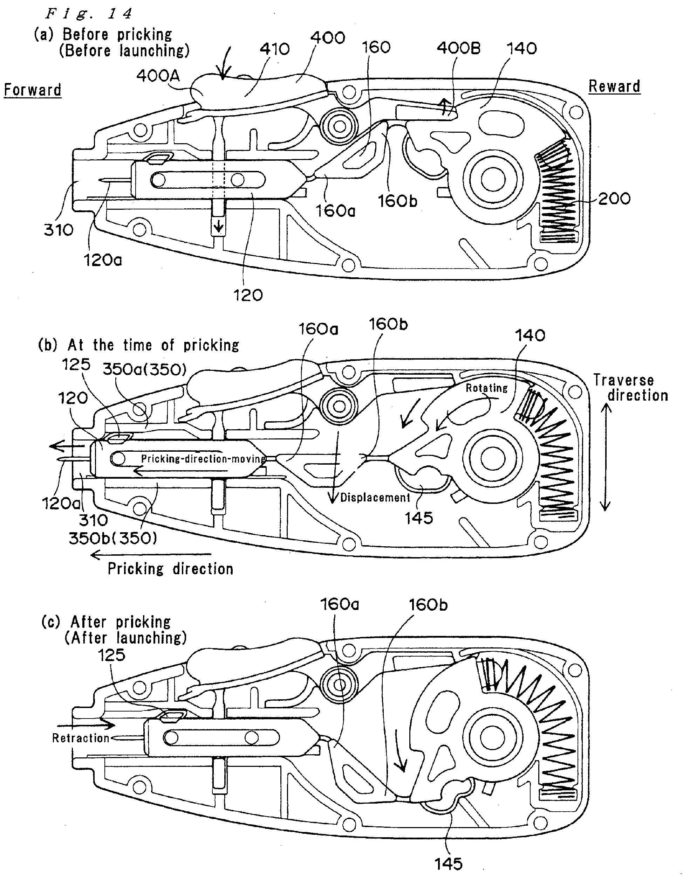

Fig. 14(a) is a cross-sectional view showing the lancet device 600 before the pricking and before the launching;
Fig. 14(b) is a cross-sectional view showing the lancet device 600 at the time of the pricking;
Fig. 14(c) is a cross-sectional view showing the lancet device 600 after the pricking and after the retraction of the needle.

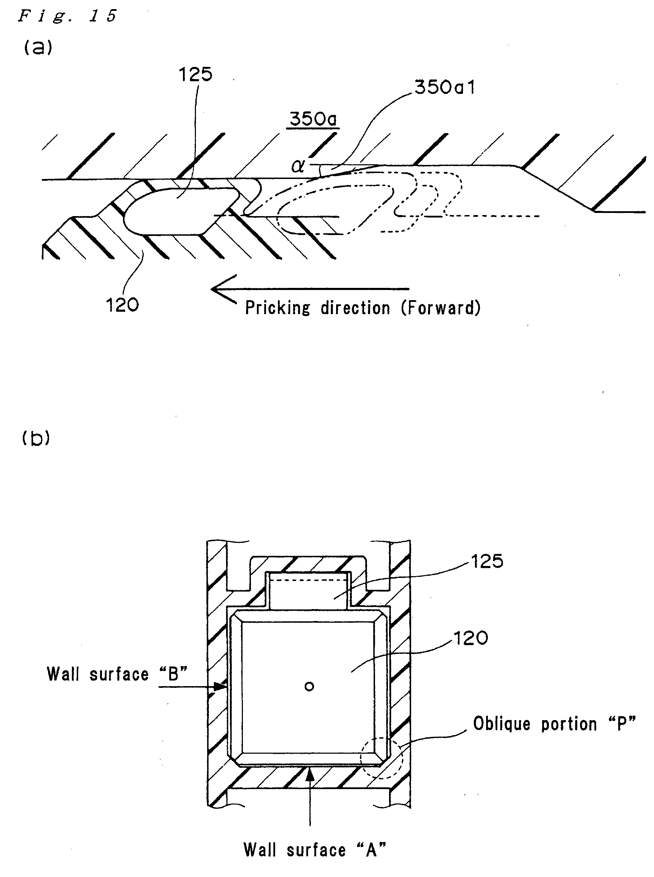

Fig. 15(a) is a schematic cross-sectional view showing an embodiment wherein an elastic part of a needle shaft component slides on a taper section;
Fig. 15(b) is a cross-sectional view showing an embodiment wherein the needle shaft component slides on a guide rail.

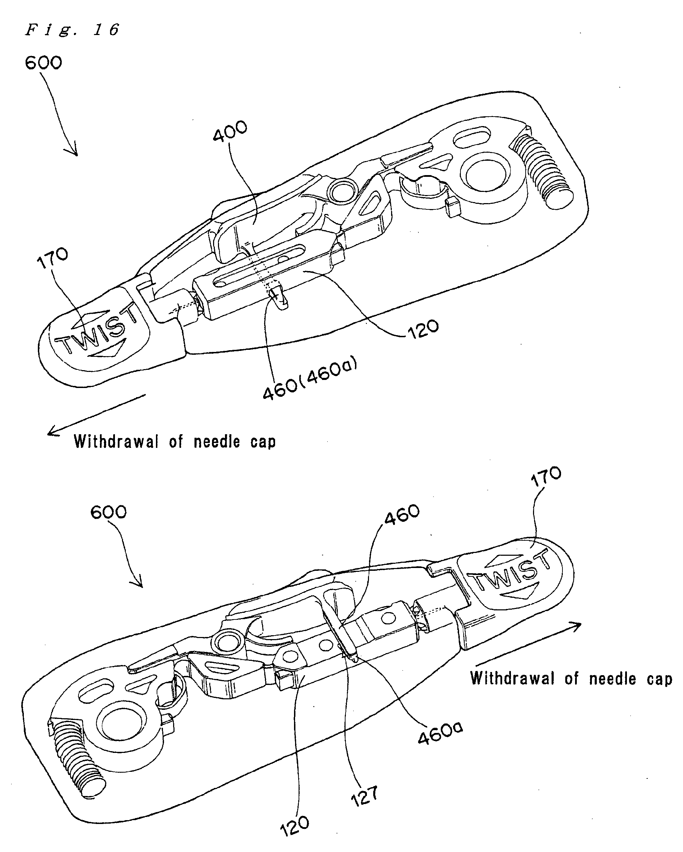

Fig. 16 is a perspective view of the lancet device 600, showing a movement-inhibiting component of a trigger part.

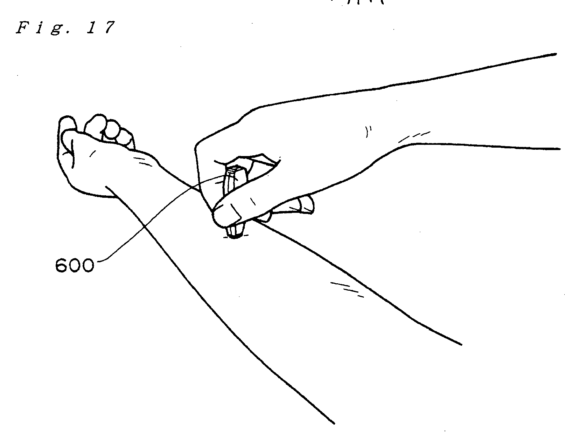

Fig. 17 is a perspective view exemplarily showing the use of the lancet device 600 according to embodiments of the present invention.

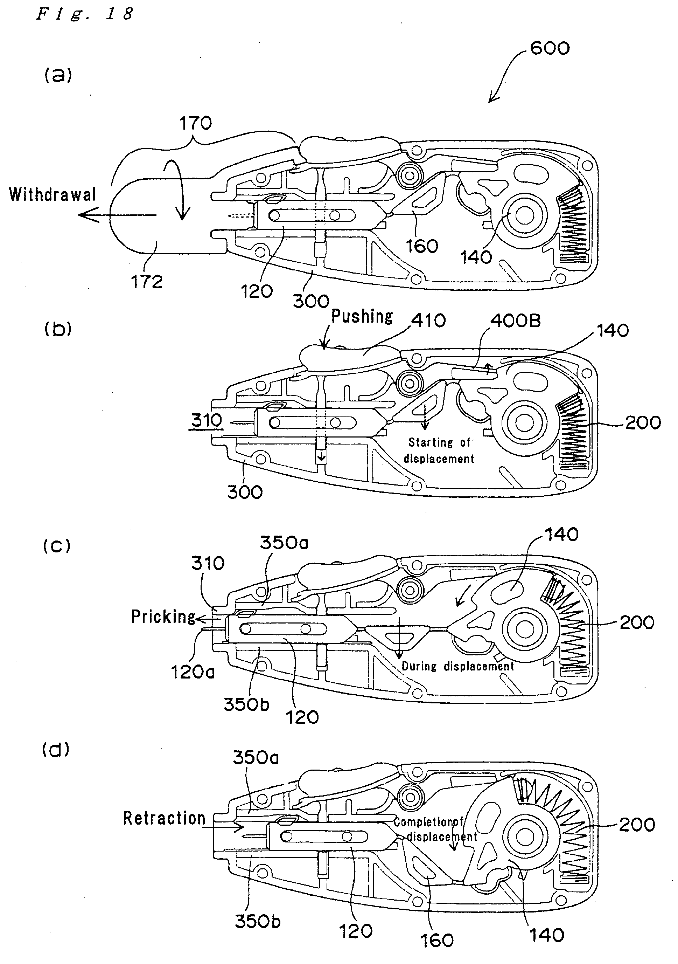

Figs. 18(a) to 18(d) are schematic cross-sectional views exemplarily showing the changes in the lancet device 600 of embodiments of the present invention over time upon using thereof.

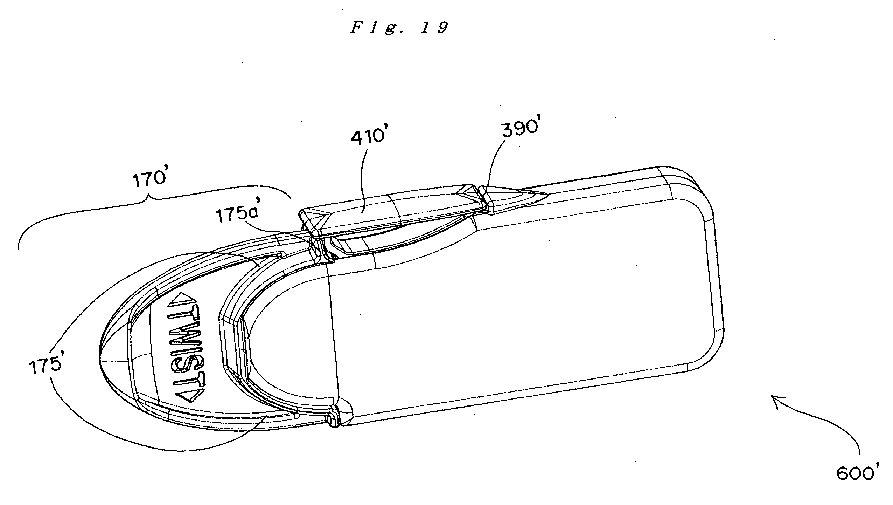

Fig. 19 is a perspective view showing an appearance of a lancet device 600' according to embodiments of the present invention.

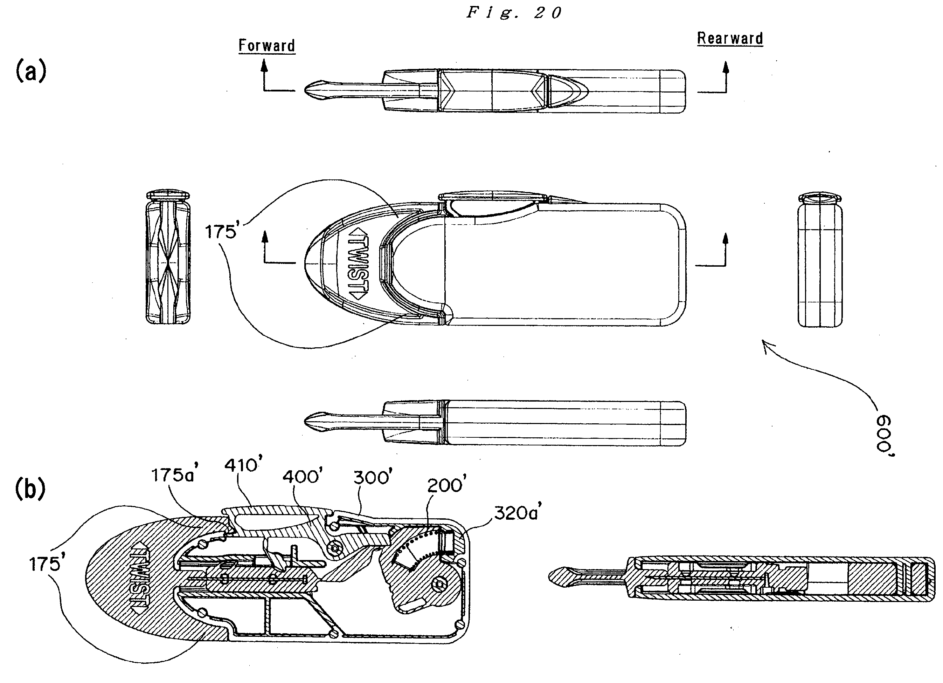

Fig. 20 shows an appearance view, a side view and a cross-sectional view of the lancet device 600' according to embodiments of the present invention.

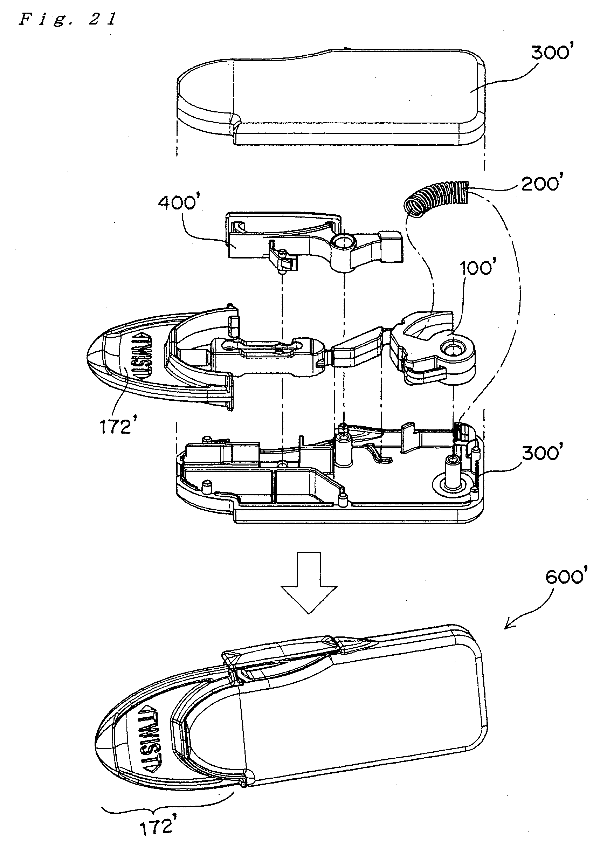

Fig. 21 shows an exploded perspective view and an appearance perspective view of the lancet device 600' according to embodiments of the present invention.

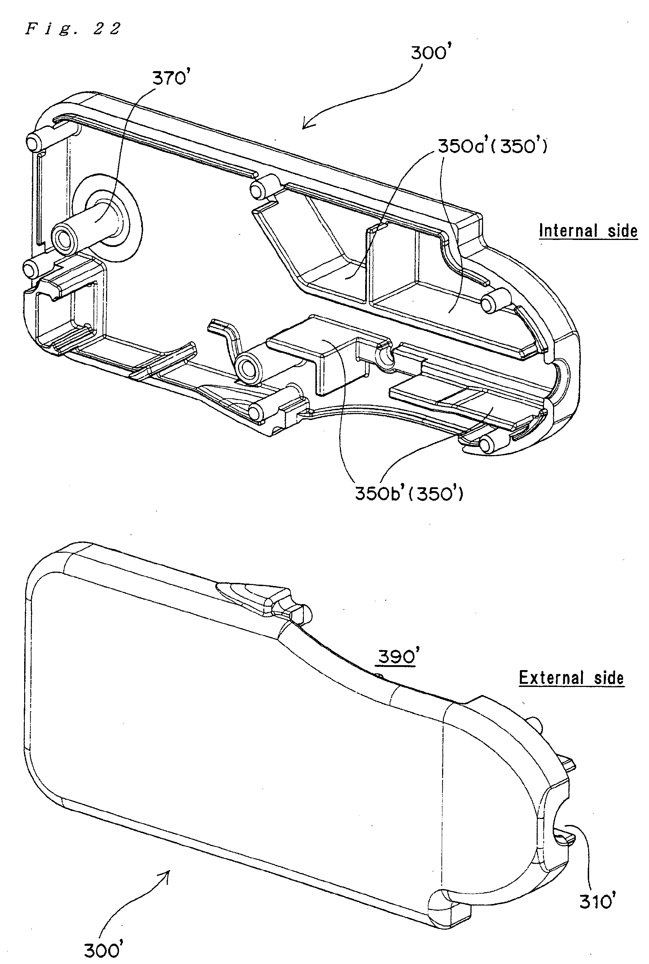

Fig. 22 is a perspective view of a housing (one of half parts of the housing) used for the lancet device 600'.

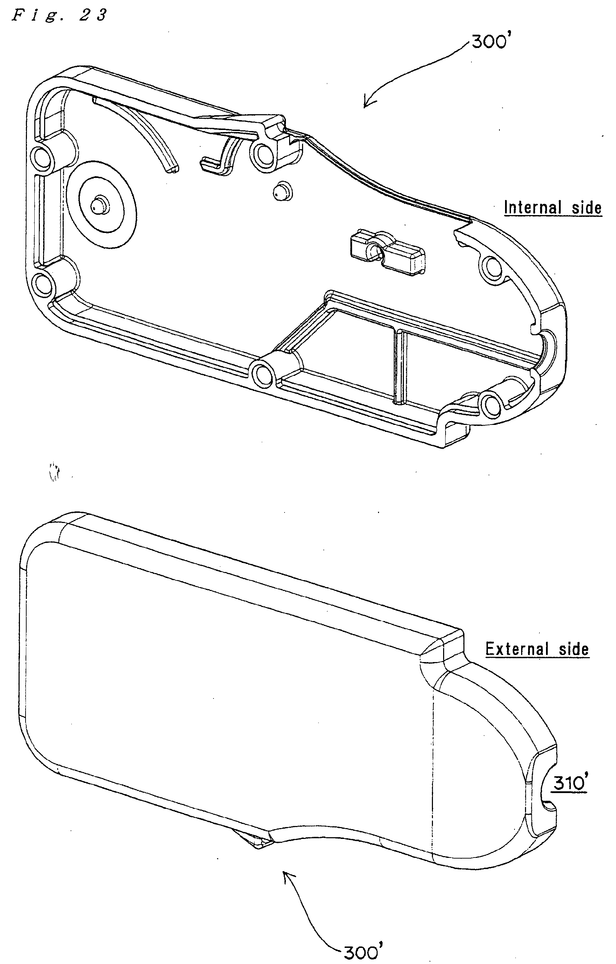

Fig. 23 is a perspective view of the housing (the other of the half parts of the housing) used for the lancet device 600'.

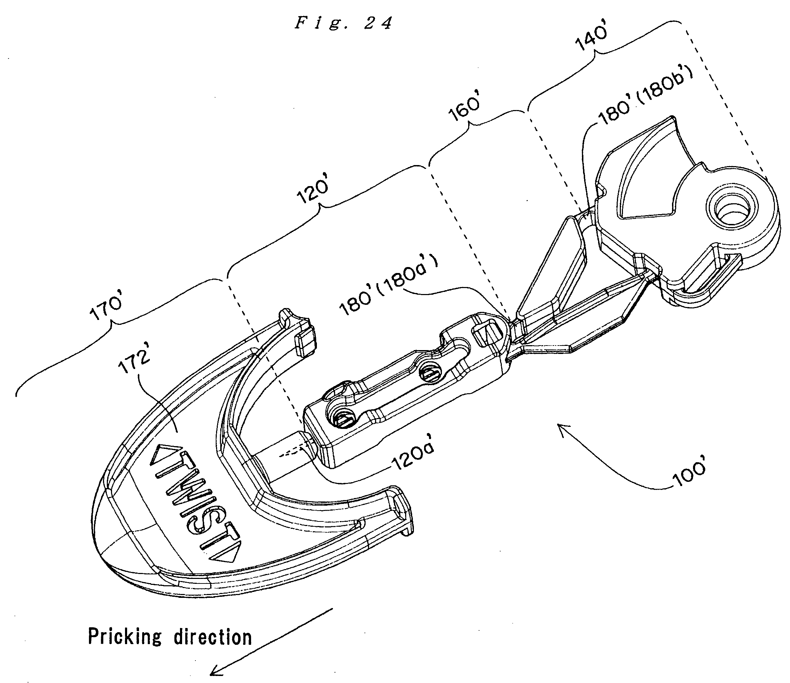

Fig. 24 is a perspective view of the pricking part (lancet pricking part) used in the lancet device 600'.

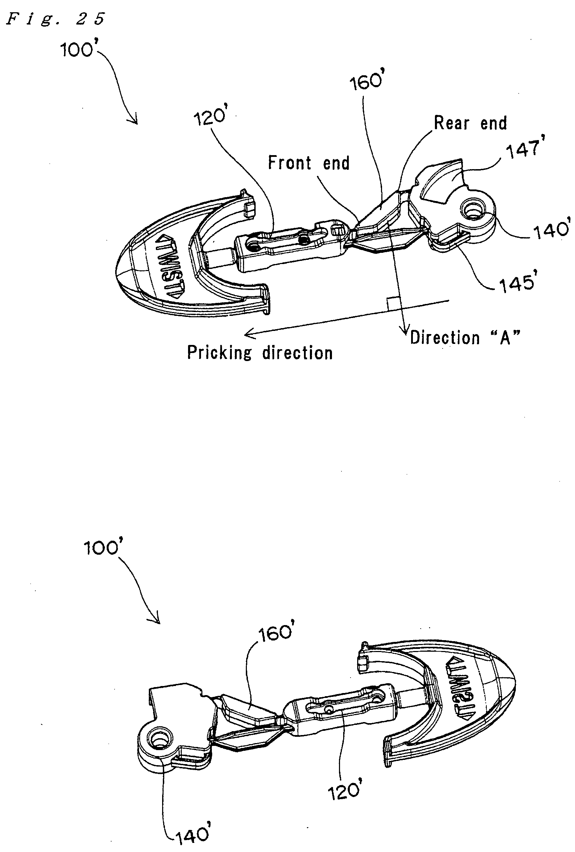

Fig. 25 is a perspective view of the pricking part (lancet pricking part) used in the lancet device 600'.

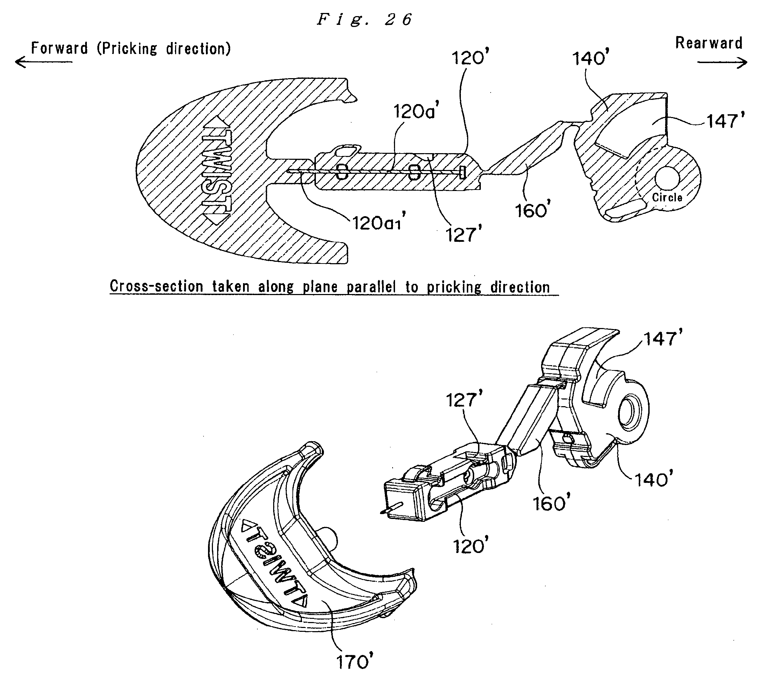

Fig. 26 shows a cross-sectional view and a perspective view of the pricking part (lancet pricking part) used in the lancet device 600'.

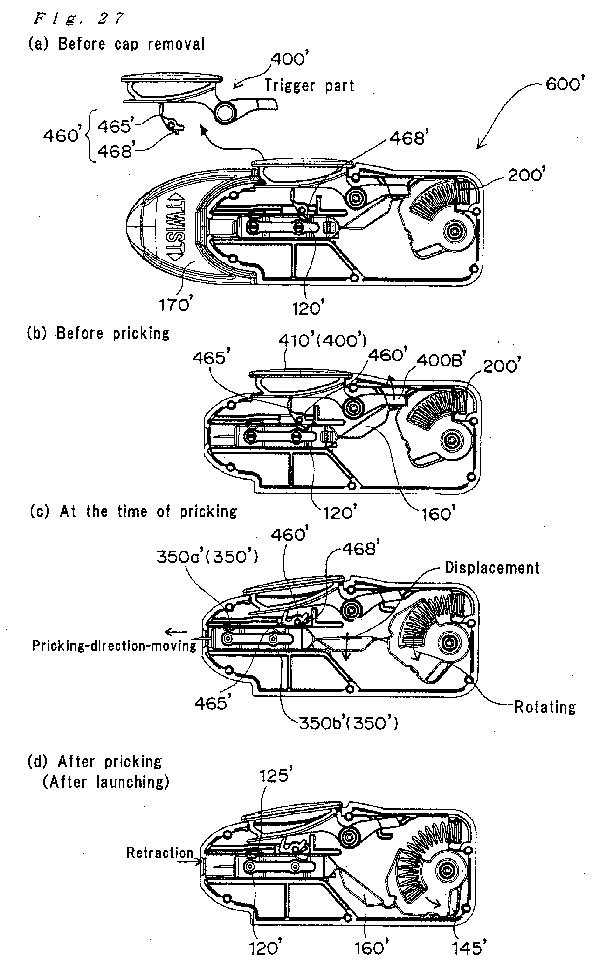

Fig. 27(a) is a cross-sectional view showing the lancet device 600' before a removal of a cap;
Fig. 27(b) is a cross-sectional view showing the lancet device 600' before the pricking and before the launching;
Fig. 27(c) is a cross-sectional view showing the lancet device 600' at the time of the pricking; Fig. 27(d) is a cross-sectional view showing the lancet device 600' after the pricking and after the retraction of the needle.

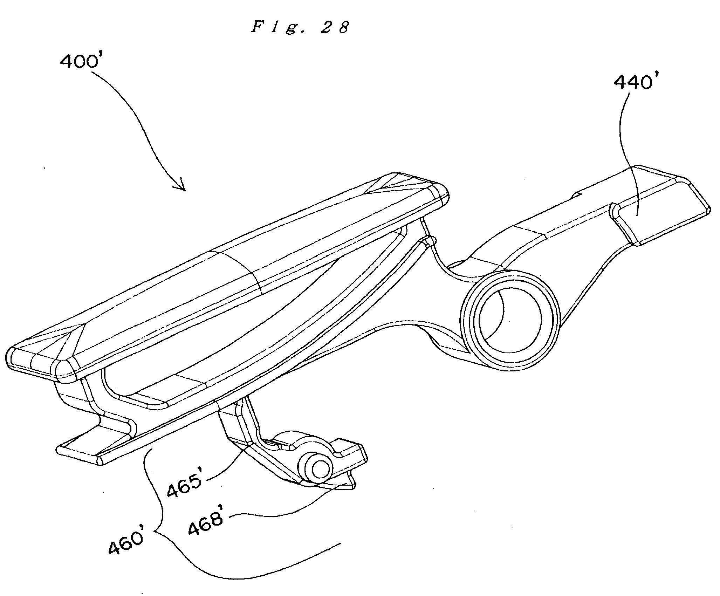

Fig. 28 is a perspective view of a trigger part used in the lancet device 600'.

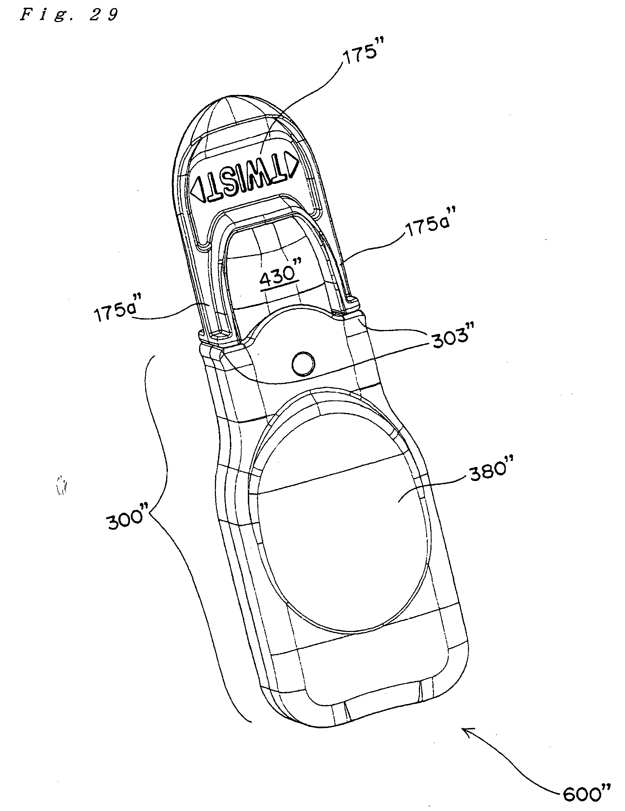

Fig. 29 is a perspective view showing an appearance of a lancet device 600" according to embodiments of the present invention.

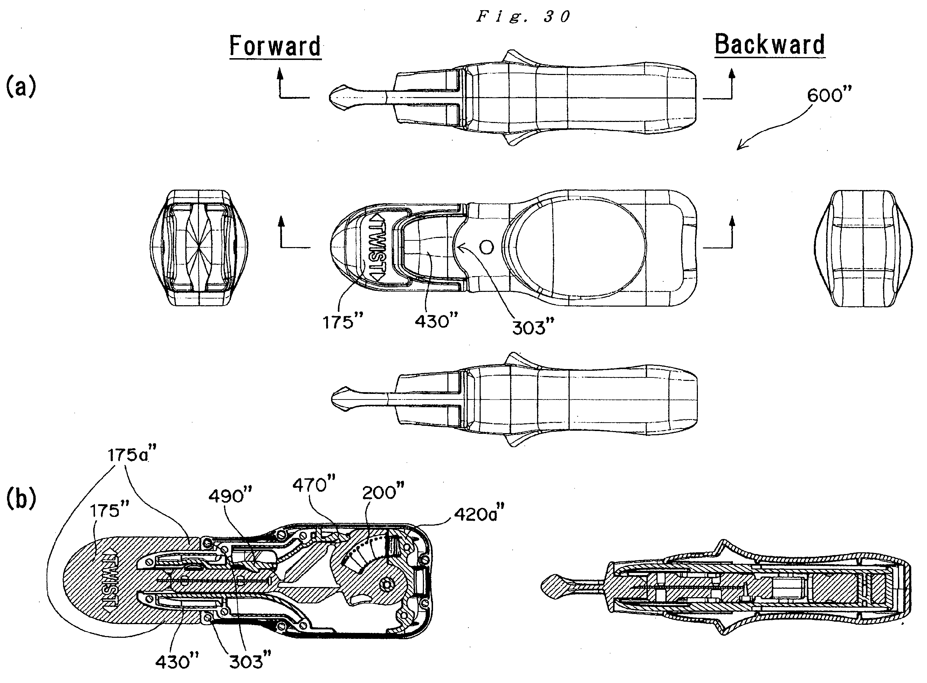

Fig. 30 shows an appearance view, a side view and a cross-sectional view of the lancet device 600" according to embodiments of the present invention.

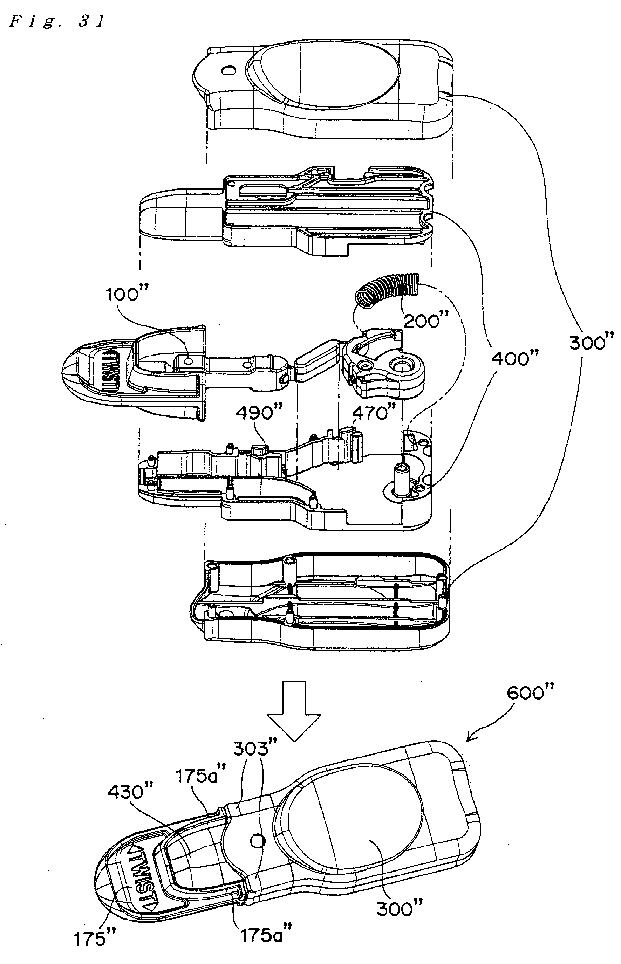

Fig. 31 shows an exploded perspective view and an appearance perspective view of the lancet device 600" according to embodiments of the present invention.

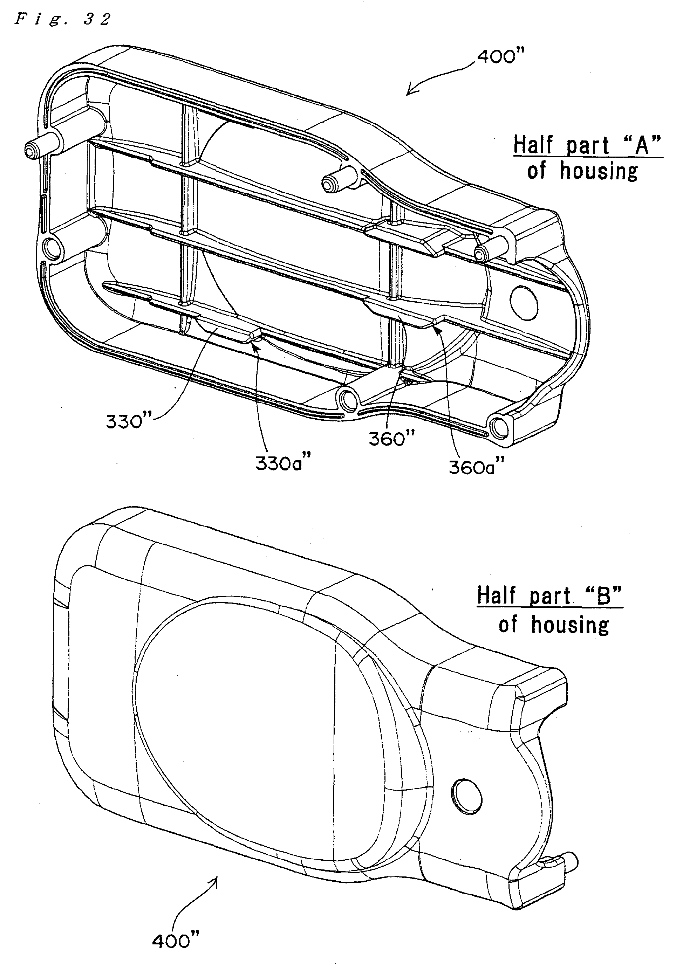

Fig. 32 is a perspective view of a housing used for the lancet device 600".

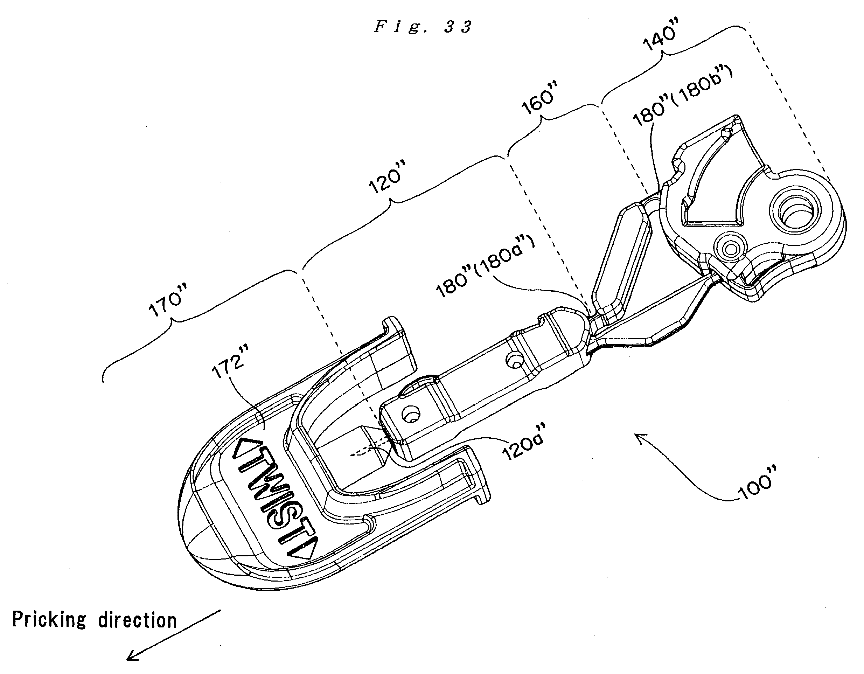

Fig. 33 is a perspective view of the pricking part (lancet pricking part) used in the lancet device 600".

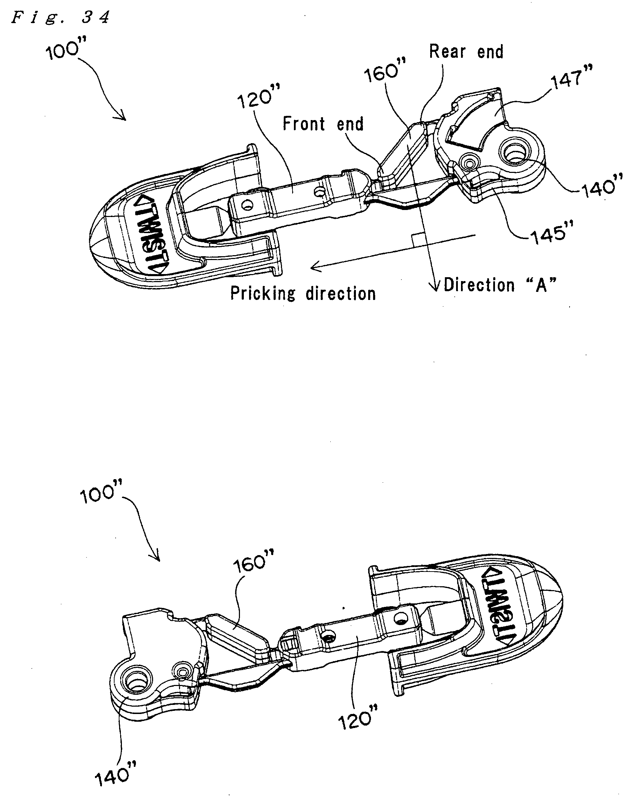

Fig. 34 is a perspective view of the pricking part (lancet pricking part) used in the lancet device 600".

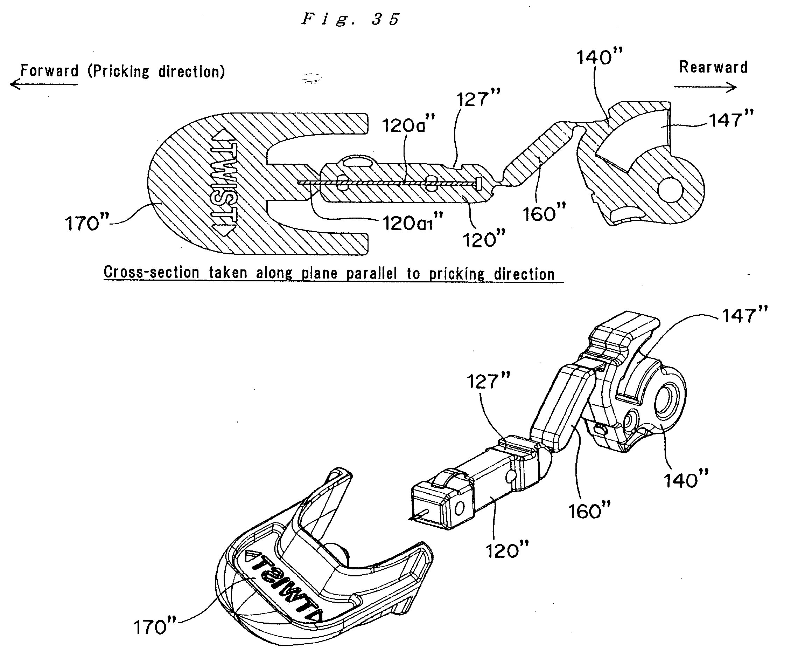

Fig. 35 shows a cross-sectional view and a perspective view of the pricking part (lancet pricking part) used in the lancet device 600".

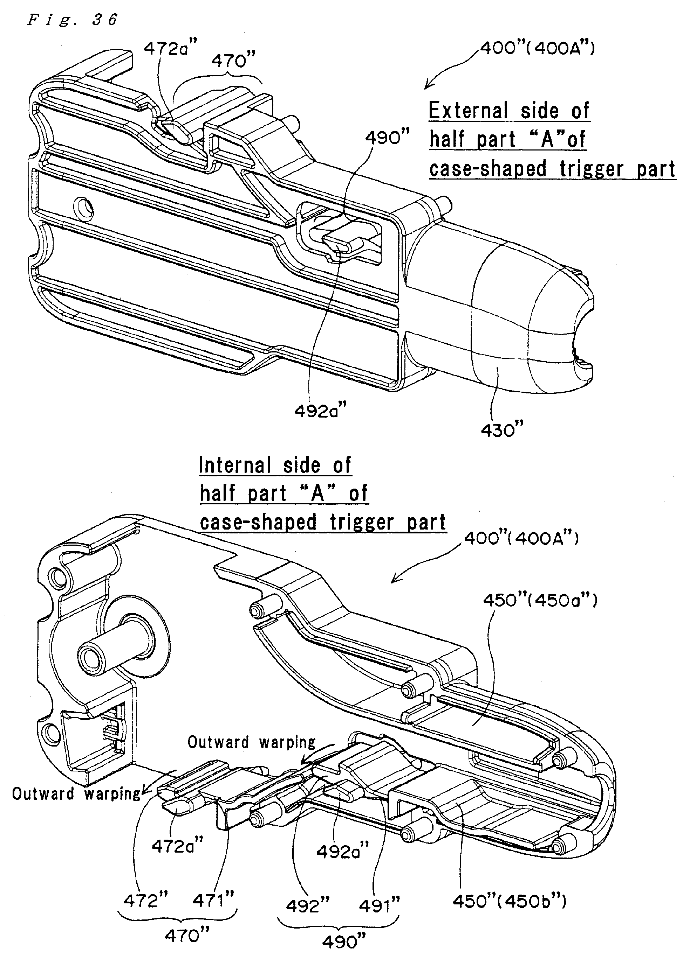

Fig. 36 is a perspective view of a case-shaped trigger part (one of halved member of the case-shaped trigger part) used for the lancet device 600".

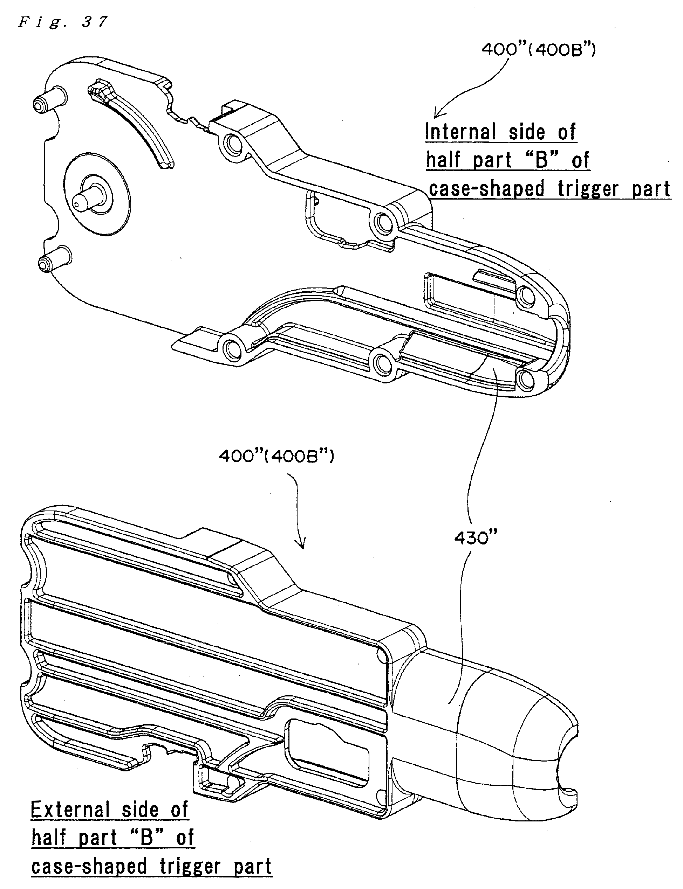

Fig. 37 is a perspective view of a case-shaped trigger part (the other of halved member of the case-shaped trigger part) used for the lancet device 600".

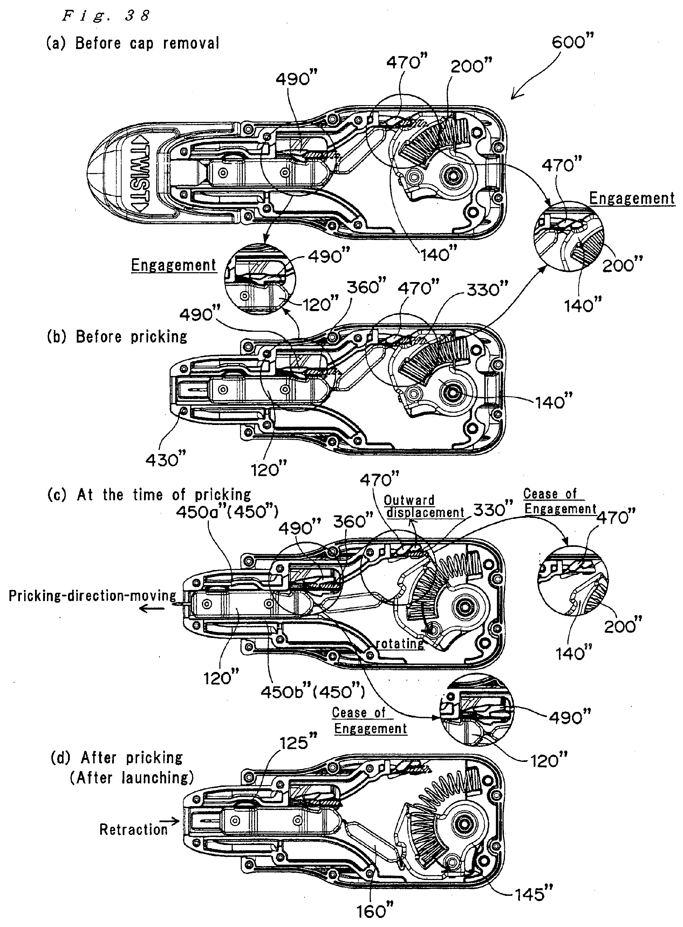

Fig. 38(a) is a cross-sectional view showing the lancet device 600" before a removal of a cap;
Fig. 38(b) is a cross-sectional view showing the lancet device 600" before the pricking and before the launching;
Fig. 38(c) is a cross-sectional view showing the lancet device 600" at the time of the pricking;
Fig. 38(d) is a cross-sectional view showing the lancet device 600" after the pricking and after the retraction of the needle.

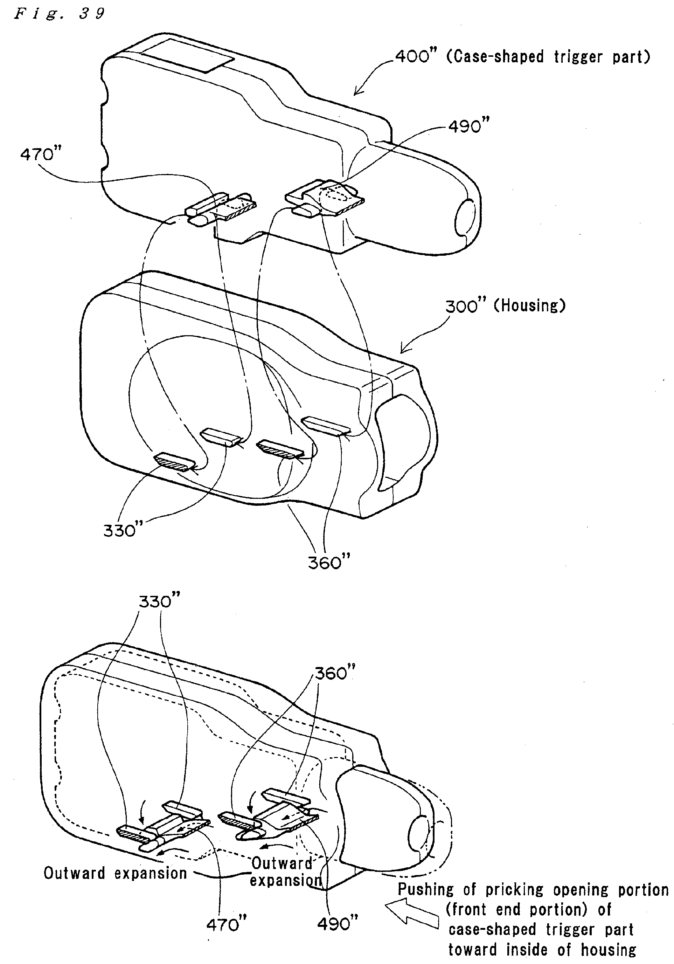

Fig. 39 is a schematic diagram for explaining mechanisms of the lancet device 600", showing a rotation starting mechanism of a driving shaft component and a mechanism for releasing a movement-inhibiting function in needle shaft component.

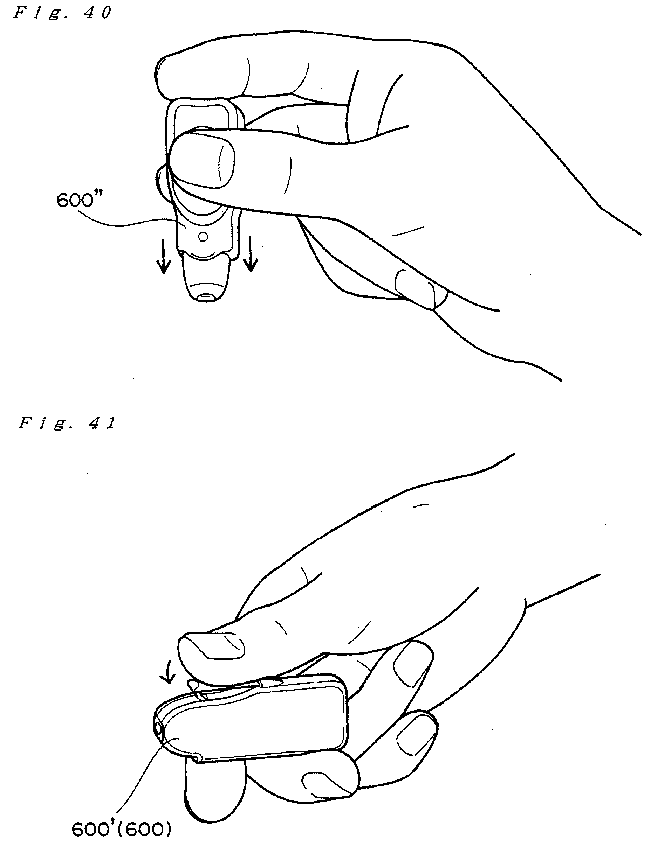

Fig. 40 is a perspective view exemplarily showing the use of the lancet device 600" (600) according to embodiments of the present invention.
Fig. 41 is a perspective view exemplarily showing the use of the lancet device 600' (600) according to embodiments of the present invention.

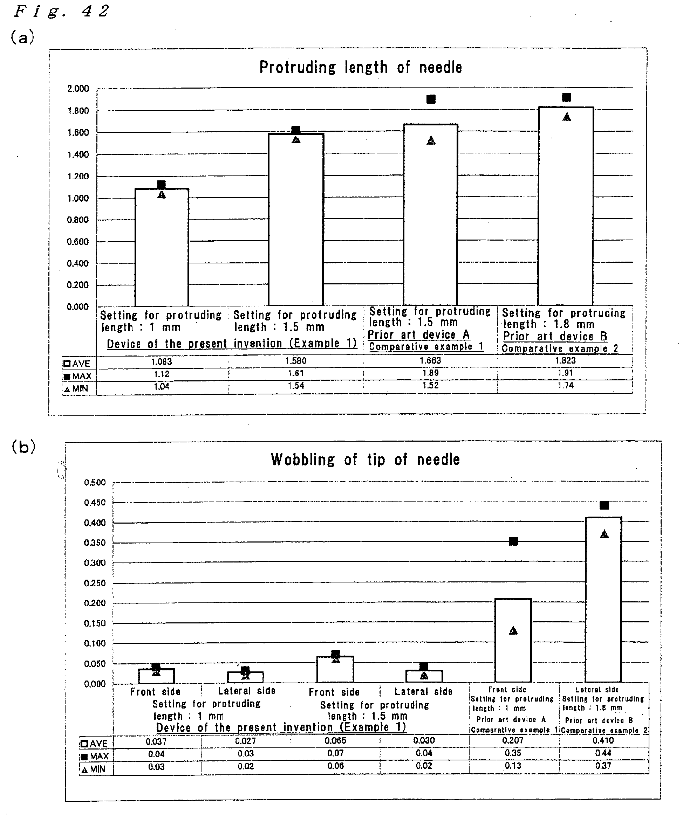

Fig. 42 is graphs showing the result of examples.
Fig. 43 is a perspective view showing an appearance of a lancet assembly (Prior Art).
Fig. 44 is a perspective view showing an appearance of an injector (Prior Art).
Fig. 45 is a perspective view showing an appearance of a lancet (Prior Art).
Fig. 46 is a perspective view showing a lancet of Fig. 45, cut away in half so as to make it easy to understand the inside of the lancet (Prior Art).
Fig. 47 is a perspective view showing the state before a lancet assembly is loaded into an injector (Prior Art).
Fig. 48 is a perspective view showing the state in which a lancet is held by the tip of a plunger upon loading a lancet assembly (Prior Art).
Fig. 49 is a perspective view showing the state of completion of loading a lancet assembly wherein a plunger cannot be retracted any more (Prior Art).
Fig. 50 is a perspective view sowing the state in which a lancet cap has been removed and thus a lancet is ready for pricking (Prior Art).

### BEST MODES FOR CARRYING OUT THE INVENTION

A lancet device according to embodiments of the invention will be described in detail below with reference to the accompanying drawings.

The term "direction" as used throughout the claims and description is defined as follows: The direction in which the pricking needle is launched for pricking is regarded as a "forward" direction. The reverse direction thereto is regarded as a "backward"/"rearward" direction. Not only "forward"/"backward", but also "traverse direction" and other directions are illustrated in the drawings. It should be noted that the "forward direction" corresponds to "pricking direction" in which direction the pricking needle moves toward the blood sampling region.

### <Basic Structure of Lancet Device>

### (Basic Structure)

Figs. 2 to 5 illustrate a lancet device 600 according to embodiments of the invention. Fig. 2 illustrates an appearance diagram of the lancet device 600, and Fig. 3 illustrates an appearance diagram of the lancet device 600, and a cross-sectional view thereof showing the interior of the lancet device 600. Fig. 4 illustrates exploded and development diagrams of the lancet device 600. Fig. 5 illustrates a transparent diagram of the lancet device 600. As shown in such diagrams (particularly as shown in Fig. 4), the lancet device 600 is mainly composed of "lancet pricking part 100", "pressing part 200", "housing 300" and "trigger part 400".

As shown in Figs. 3 to 5, the lancet device 600 has such a structure that the lancet pricking part 100, the pressing part 200 and the trigger part 400 are accommodated within the housing 300. Specifically, as shown in Fig. 5, the lancet pricking part 100 (except for a holding portion 172 of a needle cap) and the pressing part 200 are accommodated in the housing 300 such that the pressing part 200 is sandwiched between the rear end of the lancet pricking part 100 and the inner wall of the housing 300. More specifically, as shown in Fig. 3(b), the pressing part 200 is arranged in the housing 300 such that one end of the pressing part 200 is attached to the rear end 133 of the lancet pricking part 100, whereas the other end of the pressing part 200 is in contact with a contact surface 320a provided in the rear end side of the housing 300. Particularly at a point in time before pricking, the pressing part 200, which is arranged in the housing 300, is in a compressed state between the "contact surface 320a provided in the rear end side of the housing 300" and the "rear end 133 of the lancet".

The lancet device has so small thin size as a whole that it has a compact shape. For example, a thickness "T" of the device, which is as shown in Fig. 2, is in the approximate range of 4 mm to 9 mm (e.g., about 6 mm) on average.

In the following, parts or components regarding the lancet device 600 will be described.

### (Housing)

Figs. 6 and 7 are exploded and development diagrams of the housing 300 used in the lancet device 600. As shown in Figs. 6 and 7, the housing 300 preferably has a flat shape as a whole. The thickness dimension of the housing 300 is relatively small. For example, a thickness "T₀" of the housing 300, which is as shown in Fig. 6, is preferably in the approximate range of 2 mm to 4.5 mm (for example, about 3 mm). Other dimensions of the housing, for example, various dimensions shown in Fig. 6 (i.e., L, H₁, H₂, D) can be as follows: L = 22.5 mm to 67.5 mm (for example, about 45 mm), H₁ = 4 mm to 12 mm (for example, about 8 mm), H₂ = 7.5 mm to 22. 5 mm (for example, 15mm), and D= 2 mm to 6 mm (for example, about 4 mm). The shape of the housing 300 is not necessarily limited to a flat form. Thus, the shape of the housing 300 may be a rectangular tube form (box form) or a cylindrical form, for example. The holder 300 may be formed of any kind of resin materials as long as such resin can be used for lancet in general (especially, a holder of the general lancet).

As shown in Figs. 6 and 7, the housing 300 is provided with a pricking opening 310. The opening 310 is a portion applied to the region (e.g., finger) to be pricked when the pricking operation is performed. Upon the pricking, the pricking needle is exposed through the opening 310.

A guide rail 350 is provided on an inner wall of the housing 300. The guide rail 350 works in cooperation with a needle shaft component of the lancet pricking part. As shown in Fig. 6, the guide rail 350 of the housing is composed of two opposed elongate portions (350a, 350b) which protrude vertically with respect to the inner wall of the housing. The guide rail 350 allows a sliding movement of the needle shaft component 120. Specifically, when the needle shaft component 120 of the lancet pricking part 100 moves in the pricking direction and an opposite direction thereto, the needle shaft component 120 slides while being in contact with the guide rail, which leads to an achievement of the linearity of the needle movement (see Fig. 14 to be described later).

A boss 370 is provided on an inner wall of the housing 300. The boss 370 serves as a positioning member of a driving shaft component of the lancet pricking part. As shown in Fig. 6, it is preferred that the boss 370 has a cylindrical shape, and also it protrudes vertically from the inner wall of the housing. A driving shaft component 140 of the pricking part 100 has a hollow center portion therein into which the boss 370 of the housing is fitted. With such an arrangement, the driving shaft component 140 of the pricking part 100 is capable of rotating with its center being as an axis of rotation (i.e., while the boss 370 serves as a rotation axis of the driving shaft component 140).

### (Lancet Pricking Part)

The lancet pricking part 100, i.e., pricking part 100 used in the lancet device 600 is illustrated in Figs. 8 to 10. In Particular, Fig. 9 illustrates the lancet pricking part 100 in different angles from each other. Similarly to the housing 300, the lancet pricking part 100 has such a small size as to be accommodated in the housing 300 (except for the needle cap of the lancet pricking part 100). For example, the dimensions (L₀, L₁, L₂, L₃, L₄, W₁, W₂, T₁) shown in Fig. 8 are as follows: L₀ = 27.5 mm to 82.5 mm (for example, about 55 mm), L₁ = 7.5 mm to 22.5 mm (for example, about 15 mm), L₂ = 4 mm to 12 mm (for example, about 8 mm), L₃ = 7.5 mm to 22.5 mm (for example, about 15 mm), L₄ = 8.5 mm to 25.5 mm (for example, about 17 mm), W₁ = 4.5 mm to 13.5 mm (for example, about 9 mm), W₂ = 5.5 mm to 16.5 mm (for example, about 11 mm), and T₁ = 1.5 mm to 4.5 mm (for example, about 3 mm).

The lancet pricking part 100 is composed of a plurality of shafts (which mainly serve as movement or displacement components), and a plurality of joint components for jointing the shafts together. Specifically, as shown in Figs. 8 to 10, the lancet pricking component 100 comprises "needle shaft component 120 equipped with a pricking needle 120a", "driving shaft component 140", and "intermediate shaft component 160 positioned between the components 120 and 140". The needle shaft component 120, the driving shaft component 140 and the intermediate shaft component 160 are respectively coupled to each other via joint components 180. More specifically, the needle shaft component 120 and the intermediate shaft component 160 are coupled to each other by a joint component 180a, whereas the intermediate component 160 and the driving shaft component 140 are coupled to each other by the joint component 180b. In other words, the joint component 180a is provided between the needle shaft component 120 and the intermediate shaft component 160, whereas the joint component 180b is provided between the intermediate component 160 and the driving shaft component 140. To put it another way, the lancet pricking part 100 is composed of "needle member 120 equipped with the pricking needle 120a", "driving member 140 capable of moving to supply a driving force for pricking" and "intermediate member 160 provided between the needle member 120 and the driving member 140 via the joint components 180a and 180b".

It is preferred in the lancet pricking part 100 that the needle shaft component 120, the driving shaft component 140, the intermediate shaft component 160 and the joint components 180 therebetween are formed integrally. That is, it is preferred that the needle shaft component 120, the driving shaft component 140, and the intermediate shaft component 160 are not in an assembled state with each other by fitting, screw or the like, and that they form an integral single part via the joint components 180. As shown in Figs. 8 to 10, the lancet pricking part 100 has such a structure that the needle shaft component 120, the driving shaft component 140, the intermediate shaft component 160, and the joint components 180 therebetween are disposed substantially in the same plane. In other words, the needle shaft component 120, the driving shaft component 140, the intermediate shaft component 160, and the joint components 180 therebetween are not disposed so as to be superimposed over each other in the lancet pricking part 100. The joint component 180 is preferably flexible (that is, preferably, the joint component 180 can be flexed due to an external force). For example, it is preferred that the joint components 180 of the integrally formed lancet pricking part 100 are flexible. It is particularly preferred that the joint component 180 is flexible such that the needle shaft component 120 is displaced and moved due to the displacement of the intermediate shaft component 160 caused by the rotation of the driving shaft component 140. From another viewpoint, it is preferred that the joint component 180 has flexibility so that a rear end of the intermediate shaft component 160 moves in the direction perpendicular to the pricking direction of the needle shaft component 120 such that a front end of the intermediate shaft component 160 serves as an axis of the movement of the rear end. In a case where the lancet pricking part is in an integrally formed shape or in an integral single part, the joint component 180 is preferably in a thin form (small thickness form) of the pricking part. For example, a thickness dimension "t" of the joint component, which is shown in Fig. 8, is preferably in the approximate range of 0.3 mm to 1.7 mm, and more preferably in the approximate range of 0.6 mm to 1.4 mm (for example, about 1 mm).

Suitable materials for the lancet pricking part 100 (i.e., suitable materials for the needle shaft component 120, the driving shaft component 140, the intermediate shaft component 160 and the joint component 180 therebetween) are resin materials which are normally used for the lancet. Such resin material may be polyethylene or polypropylene, for example. It is preferred that the resin material for the pricking part is a soft resin such as at least one selected from the group consisting of low-density polyethylene, high-density polyethylene, polystyrene and elastomer.

As shown in Fig. 10, the needle shaft component 120 of the lancet pricking part 100 is provided with the pricking needle 120a. The pricking needle 120a may be a needle made of metal, for example. Suitable metals for the pricking needle 120a are any metal which is normally used for a pricking needle of the lancet. For example, the pricking needle 120a may be a stainless-steel needle. As shown in the cross sectional view of Fig. 10, the pricking needle 120a is situated in both the resin needle shaft component 120 and the resin needle cap 170 wherein the tip 120a1 of the pricking needle is covered with the lancet cap 170. It is preferred that the needle shaft component 120 and the lancet cap 170 are integrally connected to each other via only a small contact portion. They can be formed by inserting the pricking needle 120a into a die, in a so-called insert molding process. In this regard, the contact portion can be formed upon carrying out the insert molding process. Accordingly, the contact portion can be formed of the same resin as that of the needle shaft component 120 and the needle cap 170. The contact portion is required to be broken upon removing the needle cap. Thus, the contact portion can be referred to as a "weakened portion" or "easily broken portion". The contact portion may have a notch so that the contact portion can be easily broken. In some cases, the contact portion may be cut off in advance. No contact portion may also be provided in the needle shaft component. As long as the needle cap can be "twisted" to expose the tip 120a1 of the pricking needle 120a, the form of the contact portion is not limited to the specific one.

As shown in Figs. 8 to 10, the form of the needle shaft component 120 is not particularly limited, but it preferably has substantially a rectangular parallelepiped shape as a whole. That is, the needle shaft component preferably has a rectangular cross-sectional shape shown in Fig. 10 (that is, the shape of the cross section of the needle shaft component, taken along a plane parallel to the pricking direction, is preferably rectangular). The needle shaft component 120 with such a shape can appropriately make abutment against the guide rail 350 of the housing. When the needle shaft component 120 moves in the pricking direction and the opposite direction thereto, the needle shaft component 120 preferably slides while being in contact with the guide rail 350, which properly ensures a linear moving of the pricking needle 120a.

Turning to Figs. 8 to 10, the driving shaft component 140 of the lancet pricking part 100 is positioned at the rear-end side of the pricking part 100. The driving shaft component 140 is used such that it is in coupled to the pressing part 200 within the lancet device 600. The driving shaft component 140 can directly receive the force of the pressing part (for example, a spring force of the pressing part in a form of spring). The driving shaft component 140, which receives the force from the pressing part, can rotate about its center axis. The rotation of the driving shaft component 140 causes the intermediate shaft component 160 to be displaced, and thereby the needle shaft component 120 can move in the pricking direction and subsequently in the opposite direction thereto. That is, the driving shaft component 140 serves to transfer the force from the pressing part 200 to the intermediate shaft component 160 and further to the needle shaft component 120.

The shape of the driving shaft component 140 is not necessarily limited. It is, however, preferred that the driving shaft component 140 has the substantially disk-like shape or earlobe shape as a whole, as shown in Figs. 8 to 10. In particular, the driving shaft component 140 preferably has a circular hollow portion in its center position. That is, a cross-sectional shape of the driving shaft component 140, taken along a plane parallel to the pricking direction as shown in Fig. 10, has a circular shape in at least portion thereof. The phrase "the *cross-sectional shape* ··· *has a circular shape"* substantially covers a circle as a base shape with another shape added thereto, and/or a circle having a partially removed portion therefrom. The driving shaft component 140 in such a shape can rotate around its center due to the pressing force of the pressing part so that the force from the pressing part 200 is transferred to the intermediate shaft component 160 and then the needle shaft component 120 via the driving shaft component 140.

As shown in Figs. 8 to 10, the intermediate shaft component 160 is positioned between the needle shaft component 120 and the driving shaft component 140 in the pricking part 100. In particular, as shown in Fig. 8, the intermediate shaft component 160 is positioned between the needle shaft component 120 and the driving shaft component 140 via the joint components (180a, 180b). The intermediate shaft component 160 serves to transfer the force supplied from the driving shaft component 140 to the needle shaft component 120 when the pricking operation is carried out. Specifically, when the driving shaft component 140 is displaced due to the pressing force of the pressing part 200 such that it rotates around its center, the intermediate shaft component 160 is also displaced with the advance of the displacement of the driving shaft component 140. Further, with the advance of the displacement of the intermediate shaft component 160, the needle shaft component 120 moves in the pricking direction and then in the opposite direction thereto. As will be appreciated from the foregoing, the term *"intermediate"* of the intermediate shaft component is referred from the viewpoint of a positional or arrangement relationship, and also from the viewpoint of a transfer path of the force upon the pricking operation.

As shown in Figs. 8 to 10, the form of the intermediate shaft component 160 is not necessarily limited, but preferably it has substantially the thin trapezoidal geometry as a whole. That is, a cross-sectional shape of the intermediate shaft component, taken along a plane parallel to the pricking direction as shown in Fig. 10, is preferably trapezoidal. The intermediate shaft component 140 with such a shape can be appropriately displaced due to the rotating displacement of the driving shaft component 140. Now, the displacement of the intermediate shaft component 160 will be described in detail. As shown in Fig. 9, the intermediate shaft component 160 moves in the direction "A" perpendicular to the pricking direction of the needle shaft component 120 when the pricking operation is carried out. It is preferred that a rear end of the intermediate shaft component 160 moves in the direction perpendicular to the pricking direction of the needle shaft component such that a front end of the intermediate shaft component serves as an axis of the movement of the rear end (see Fig. 9 and Fig. 14 to be referred later). The cross-sectional shape of the intermediate shaft component is not limited to a trapezoidal one, and thus may be a triangle, rectangular or polygonal shape.

### (Pressing Part)

The pressing part 200 is illustrated in the perspective view of Fig. 11. As shown in Fig. 11, the pressing part 200 may be a spring. Such spring 200 indirectly serves as a means for "launching" or "firing (i.e., shooting)" of the pricking needle. In other words, the spring 200 serves to supply a thrust force for launching the pricking needle 120a (i.e., "needle shaft component 120 with the tip of the pricking needle 120a exposed") or a drive force for pricking. As will be described later, the spring 200 supplies not only the thrust/driving force for launching "the needle shaft component 120 with the tip of the pricking needle 120a exposed", but also another force for pulling the needle shaft component so that it moves back in the direction opposite to the pricking direction at a point in time after the pricking. Such spring 200 is provided while being in a compressed state between "contact surface 320a at the rear end side of the housing" and "rear end 133 of the lancet pricking part", which is described above (see Fig. 3). The spring 200 is preferably made of metal. For example, the spring 200 may be a metal coil spring, a metal torsional spring or a leaf spring. The size of the spring 200 is not necessarily limited to a specific one, and thus may be any one as long as the spring can be accommodated in the housing 300. For example, the dimensions (L₅, D₁) of the spring which is not in a compressed state as shown in Fig. 11 may be as follows: L₅ = 10 mm to 20 mm (for example, about 15 mm), D₁ = 1.5 mm to 4. 5 mm (for example, about 3 mm).

### (Trigger Part)

The trigger part 400 used in the lancet device 600 is illustrated in Figs. 12 to 13. In particular, Fig. 13 illustrates the different sides of the trigger part 400. Similarly to the housing 300 and the lancet pricking part 100, the trigger part 400 has such a small size as to be accommodated in the housing 300 (except for a front-sided upper portion 410a of the trigger part). Specifically, the trigger part 400 is accommodated in the housing such that the front-sided upper portion 410a of the trigger part is exposed from a trigger opening 390 of the housing (see Fig. 2). The trigger part 400 may have, for example, the following dimensions (L₆, W₃, W₄, H₃) as shown in Fig. 12: L₆ = 12.5 mm to 37.5 mm (for example, about 25 mm), W₃ = 2.25 mm to 6.75 mm (for example, about 4.5 mm), W₄ = 2 mm to 6 mm (for example, about 4 mm), and H₃ = 4.5 mm to 13.5 mm (for example, about 9 mm). Such trigger part 400 may be made of the same material as the lancet pricking part 100 (for example, polyethylene, polypropylene, or the like).

As shown in 12 to 13, it is preferred that the trigger part 400 has an elongate component 440 which extends backward, and also it has a pushing portion 410 which is located at the front side of the trigger part, the pushing portion 410 being to be pushed into the housing by a finger or the like for pricking. In the lancet device 600, a rear end 400B of the trigger part 400 is in abutment against the driving shaft component 140 of the pricking part, and thereby the spring 200 coupled to the driving shaft component 140 is kept compressed (see Fig. 5). The trigger part 400 is pivotally provided on the housing inner wall via its body opening 460, and thus the trigger part 400 is capable of being displaced around the position of the body opening 460. Specifically, when the pushing portion 410 of the trigger part 400 is pushed toward an inside of the housing, a front end 400A of the trigger part 400 moves inwardly whereas the rear end 400B of the trigger part 400 moves outwardly (see Fig. 5). Thus, when the front side 410 of the trigger part is pushed toward the inside of the housing for the purpose of carrying out the pricking, the contract between the rear end 400B of the trigger part and the driving shaft component 140 of the pricking part cannot be maintained any more, and as a result of this the driving shaft component 140 is pressed in the rotation direction by the spring 200.

### «Entire Structure and Function of Lancet Device»

### <Engagement of Driving Shaft Component>

As shown in Fig. 14(a), the lancet device 600 is configured such that the driving shaft component 140 of the lancet pricking part 100 is in engagement with the rear end 400B of the trigger part 400 to maintain the compressed state of the spring 200. That is, in the lancet device at a point in time before the pricking operation, the driving shaft component 140 is subjected to the force for moving the shaft component 140 in the rotation direction by the "compressed spring 200".

When the pushing portion 410 of the trigger part 400 is pressed toward the inside of the housing for the purpose of carrying out the pricking operation, the rear end 400B of the trigger part 400 moves outwardly to cause a ceasing of the engagement between the rear end 400B of the trigger part and the driving shaft component 140. The ceasing of such engagement allows the driving shaft component 140 to be rotated around its rotation axis due to the pressing action of the spring 200. That is, the driving shaft component 140 at a point in time after the ceasing of the engagement is displaced by the pressing action of the spring 200 as shown in Fig. 14(b). The advance of the displacement of the driving shaft component 140 causes the intermediate shaft component 160 to be displaced, and thereby the needle shaft component 120 can move in the pricking direction and subsequently in the opposite direction thereto (see Figs 14(b) and 14(c)). As the change in stage over time shown in Fig. 14, the intermediate shaft component 160 is displaced in the direction in a traverse direction of the device. Namely, the intermediate shaft component 160 moves in the direction perpendicular to the pricking direction of the needle shaft component 120. More specifically, during the displacement of the intermediate shaft component 160 of the pricking part, the rear end 160b of the intermediate shaft component 160 moves in the direction perpendicular to the pricking direction of the needle shaft component 120 while the front end 160a of the intermediate shaft component 160 serves as a moving axis of the rear end 160b. In other words, the rear end 160b of the intermediate shaft component 160 positively moves in the traverse direction of the device, whereas the front end 160a of the intermediate shaft component 160 substantially maintains its position. This means that the displacement direction of the intermediate shaft component 160 (i.e., moving direction of the component 160) and the pricking direction of the needle shaft component 120 (i.e., moving direction of the component 120) are not in the same line as each other in the device 600.

As can be seen from the embodiments shown in Figs. 14(a) to 14(c), the lancet device 600 of embodiments of the present invention has such a function that the rotation of the driving shaft component 140 of the pricking part 100 causes the intermediate shaft component 160 of the pricking part to be displaced, and thereby allowing the needle shaft component 120 of the pricking part 100 to be moved in the pricking direction. Because of such function, the lancet device 600 can produce a large force for moving the needle shaft component 120 in the pricking direction. Thus, even while the needle shaft component 120 is in strong abutment against the guide rails (350a, 350b), the needle shaft component 120 can move in the pricking direction, which appropriately ensures the linearity of the needle shaft component 120 (see Fig. 14(b)). That is, the needle shaft component 120 of the lancet device can forcefully perform a sliding movement in the pricking direction, even when the needle shaft component 120 is largely pressed against the guide rails (350a, 350b), causing a large sliding resistance between the body of the needle shaft component 120 and the surface of the guide rails (350a, 350b). This means that the lancet device according to embodiments of the invention appropriately achieves the linearity of the needle shaft component based on the guide rails.

Now, the linearity of the needle shaft component will be described in more detail. It is preferred that the side face of the needle shaft component 120 is provided with an elastic portion 125 having a hollow structure. When the needle shaft component 120 moves in the pricking direction and in the opposed direction thereto, at least the elastic portion 125 preferably moves while being in contact with the guide (350a) of the housing (see Fig. 14(b)). That is, when the needle shaft component 120 equipped with the pricking needle 120a moves in the pricking direction, the elastic portion 125 of the needle shaft component 120 also moves in the pricking direction in the same way. During this movement, the elastic portion 125 is preferably in contact with the guide surface of the guide rail (350a). In particular, the elastic portion is preferably brought into contact with the inner wall of the lancet housing so as to receive the force from the surface of the guide. Thus, the elastic portion 125 can absorb the shock occurred in the launched pricking needle 120a, so that the pricking needle 120a moving in the pricking direction is stabilized. In other words, the moving elastic portion 125 slides on the surfaces of the guide rails (350a, 350b), and thus the stress generated in the pricking needle 120a can be relieved by a cushion effect of the elastic portion 125, even though the pricking needle probably receives some transverse force. As a result, a linear pricking pathway of the needle can be ensured. The guide surface preferably has a tapered portion 350a₁ (that is, "slope portion"). In this case, the elastic portion 125 can effectively receive the force when it slides on the tapered portion 350a₁. That is, the moving elastic portion 125 tends to be easily warped by the tapered portion, so that the elastic portion effectively absorbs the shock of the needle shaft component 120 upon the pricking operation, which can further stabilize the pricking pathway of the pricking needle 120a.

As illustrated in the cross-sectional view of Fig. 15(b), the corner(s) of the guide may be provided with an oblique portion "P". In this case, as shown in Fig. 15(b), when the needle shaft component 120 receives the force in the traverse direction due to a counteraction to the cushion action of the elastic portion 125 or the like, the needle shaft component 120 is effectively pressed against a wall surface "A" and a wall surface "B" because of the presence of the oblique portion "P", which can more appropriately achieve the linearity of the needle shaft component, the linearity being based on the guide rails.

As can be seen from the embodiments of the lancet device of the invention shown in Figs. 14(a) to 14(c), while the compressed spring 200 is being released and extending, the needle shaft component 120 equipped with the pricking needle 120a moves in the pricking direction and subsequently moves in the opposite direction to the pricking direction. Accordingly, the device does not restrict a protruding length of the pricking needle (i.e., exposed length from the pricking opening at the time of pricking) by a collision of the pricking needle portion.

This means that "needle wobble" at the time of pricking can be appropriately avoided. As can be seen from the embodiments shown in Figs. 14(a) to 14(c), the lancet device achieves a smooth series of movements including the exposure of the pricking needle 120a from the pricking opening 310 and the retracting of the pricking needle 120a. Therefore, the movement of the pricking needle at the time of the pricking is smooth, which can suitably maintain the linearity of the needle shaft component based on the guide rails.

As can be seen from the embodiments shown in Figs. 14(a) to 14(c), the lancet device achieves both the exposure and the retracting of the pricking needle 120a while the compressed spring 200 is being released and returning to its uncompressed state. Thus, the pricking needle 120a after being once exposed never again be exposed from the pricking opening 310 due to the following reasons:
- Even when the pulsation of the spring is generated, the needle shaft component 12 has been already retracted deeply into the device at the time of such pulsation as shown in Fig. 14(c); and
- The small pulsation of the spring cannot permit a backward displacement of embodiments of the intermediate shaft component 160 in the direction opposite to the direction for pricking.

This means that the device of embodiments of the present invention can appropriately prevent a so-called "twice pricking phenomenon of the needle". It is preferred that the lancet device of embodiments of the present invention has "hollow cushion portion 145" in the driving shaft component 140. In this case, while the compressed spring 200 is being released and returning to its uncompressed state, the cushion portion 145 effectively absorbs the energy of the spring pulsation, which leads to a suppression of the spring pulsation itself (see Figs. 14(b) and 14(c)).

Now, the removal of the needle cap from the device for the purpose of carrying out the pricking operation will be described below. In the lancet device, the pricking part is not adversely affected during the removal of the needle cap from the tip of the pricking needle. Specifically, upon the removal of the needle cap, the needle shaft component is prevented from being displaced and moved.

As shown in Fig. 16, the prevention of the displacement or movement of the needle shaft component is achieved by the state in which a movement-inhibiting component 460 of the trigger part 400 and the needle shaft component 120 of the pricking part are capable of making abutment with each other. As shown in Fig. 16, a raised portion 460a of the movement-inhibiting component 460 and a recess portion 127 of the needle shaft component 120 engage with each other, and thereby the needle shaft component 120 can resist the external force caused by a pullout operation of the needle cap 170, which leads to a prevention of the adverse movement of the needle shaft component 120. When the pushing portion 410 of the trigger part 400 is pushed into the housing for the purpose of carrying out the pricking operation, the movement-inhibiting component 460 is displaced in direction of the pushing of the trigger part. The displacement of the movement-inhibiting component 460 causes a ceasing of the engagement between the raised portion 460a and the recess portion 127. Accordingly, the raised portion 460a and the recess portion 127 do not adversely affect the launching of the needle shaft component 120.

When the needle cap 170 is not removed before the pricking operation as shown in Fig. 2 for example, a backwardly-extending wing 173 of the needle cap 170 can prevent the trigger part from being pushed into the housing, which avoids an accidental launching of the pricking needle. Specifically, the backwardly-extending wing 173 of the needle cap 170 is disposed on the outer surface of the housing such that the tip 173a of the backwardly-extending wing 173 is located beneath the front end of the pushing portion 410 of the trigger part, which can prevent the pushing portion 410 from being pushed into the device. In contrast, after the needle cap 170 is removed, the backwardly-extending wing 173 is also removed. This means that the tip 173a of the backwardly-extending wing 173 is no longer located beneath the front end of the pushing portion 410 of the trigger part. As a result, the pushing portion 410 of the trigger part can be pushed toward the interior of the device. Therefore, the removal of the needle cap 170 from the pricking needle makes it possible to push the pushing portion 410 of the trigger part into the device so as to launch the pricking needle.

### «Embodiment of Use of Pricking Device»

In the following, the embodiments of use of the lancet device according to the invention will be described with reference to Figs. 17 and 18. Figs. 18(a) to 18(d) show the changes of the lancet device 600 over time in numerical order.

The lancet device 600 of the present invention at a point in time before the pricking operation is shown in Fig. 18(a). First, the needle cap 170 is removed from the lancet pricking part as shown in Figs. 18(a) and 18(b) for the purpose of carrying out the pricking operation. The removal of the needle cap 170 is performed preferably by twisting the needle cap 170. Specifically, as shown in Fig. 18(a), the needle cap 170 (in particular, the holding portion 172) is rotated to break the "contact provided between the needle shaft component 120 and the needle cap 170", and thereafter the needle cap 170 is pulled out forwardly. In other words, one hand holds the lancet device 600 from the outside, and the fingers of the other hand pull the holding portion 172 of the needle cap 170 while twisting it. As a result, the pricking needle 120a is exposed in the needle shaft component 120 within the housing 300 (see Fig. 18(b)). Then, after the pricking opening 310 of the device is applied to the predetermined region to be pricked (e.g., a fingertip), the pushing portion 410 of the trigger part is pushed toward the inside of the housing (see Fig. 18(b)). Such pushing of the trigger part causes a ceasing of the contact between the rear end 400B of the trigger part and the driving shaft component 140 of the pricking part (see Figs. 18(b) and 18(c)), which allows the driving shaft component 140 to be displaced by the pressing force of the spring 200. The displacement of the driving shaft component 140 is performed such that the component 140 rotates around its rotation axis. With the advance of the rotation of the driving shaft component 140, the intermediate shaft component 160 is displaced. The displacement of the intermediate shaft component 160 is performed such that the component 160 moves in the traverse direction of the device. Furthermore, with the advance of the displacement of the intermediate shaft component 160, the needle shaft component 120 moves in the pricking direction, and subsequently retracts in the opposite direction thereto (see Figs. 18(c) and 18(d)).

Upon the movement of the needle shaft component 120 in the pricking direction and the retraction thereof, the pricking needle 120a of the needle shaft component 120 protrudes from the pricking opening 310, and thereby the predetermined region (for example, fingertip) which is in contact with the pricking opening 310 is pricked.

As can be seen from the embodiments shown in Fig. 18(b) to 18(d), during the movement of the needle shaft component 120 in the pricking direction and the retraction thereof, the needle shaft component 120 slides in the pricking direction while being pressed against the guide rails (350a, 350b). Thus, the linearity of the needle shaft component based on the guide rails can be appropriately achieved in the lancet device 600. Likewise, as can be seen from the embodiments shown in Figs. 18(c) and 18(d), both the exposure and the retracting of the pricking needle 120a can be performed while the compressed spring 200 is released and extending to have its uncompressed state. Thus, even when the pulsation of the spring occurs, the needle shaft component 120 has been already retracted deeply into the device at the time of the pulsation as shown in Fig. 18(d). And also, the small pulsation of the spring cannot permit the backward displacement of the intermediate shaft component 160 in the direction opposite to the direction for pricking. These mean that, at a point in time after the pricking, the pricking needle 120a cannot never again protrude from the pricking opening 310.

### «Various Modified Embodiments of Lancet Device»

The present invention can be embodied in various forms. For example, the lancet device of the present invention may have the form shown in Figs. 19 to 28, or the form shown in Figs. 29 to 39. Now, these embodiments will be described (unless otherwise specified, the elements which are not described below are the same as those of the lancet device 600 described above, and the description of the same parts, components or functions will be omitted so as to avoid the redundant description. In the accompanying drawings, the same parts, components or portions as those of the above lancet device 600 are represented by the same reference numerals (except for reference numerals with " ' " or " " " attached thereto).

### (Lancet Device of Embodiment Shown in Figs. 19 to 28)

A lancet device 600' according to the embodiment of Figs. 19 to 28 has substantially the same functions as those of the above lance device 600, but mainly differs from the above lancet device 600 in form of the trigger part, arrangement of the compressed spring, and the form of the needle cap.

A trigger part 400' of the lancet device 600' has the form shown in Figs. 27 and 28. Specifically, a movement-inhibiting component 460' of the trigger part 400' has a slightly curved form. Such movement-inhibiting component 460' is deformable so as to be bent at the position of its node 465'. The deformation of the movement-inhibiting component 460' makes it possible to cease the engagement of the movement-inhibiting component 460' with a needle shaft component 120' . Specifically, as shown in Fig. 27, at a point in time before the trigger part 400' is pushed, a protrusion 468' provided in a free end of the movement-inhibiting component 460' is in engagement with a recess portion 127' of the needle shaft component 120' (see Fig. 26 as to the "recess portion 127' of the needle shaft component"), and thereby the needle shaft component 120' is prevented from being displaced and moved during the removal of the needle cap 170' (see Fig. 27(a)). When the trigger part 400' is pushed into the device at a point in time after the needle cap 170' is removed, the movement-inhibiting component 460' can be deformed at a position of the node 465' , as shown in Figs. 27(b) and 27(c). As a result, the protrusion 468' of the movement-inhibiting component 460' and the recess portion of the needle shaft component 120' are no longer in engagement with each other. After the engagement is released, nothing prevents the movement of the needle shaft component 120' , which ensures a suitable launching of the needle shaft component 120' (i.e., the pricking needle).

As for the compressed spring 200' of the lancet device 600' , the compressed spring 200' is disposed such that at least a part of the compressed spring 200' is inserted into the driving shaft component 140' . In other words, a part or most of the compressed spring 200' which is coupled to the driving shaft component 140' is accommodated within a recessed portion 147' of the driving shaft component 140' (see Figs. 25 and 26). As shown in Figs. 25 and 26, the recessed portion 147' of the driving shaft component 140' preferably has a complementary shape with respect to the entire shape of the compressed spring 200'. In this embodiment shown in Figs. 19 to 28, the entire size of the device can be reduced (especially, the longitudinal direction dimension) by an disposed length of the compressed spring 200' in the driving shaft component 140' , which leads to a more compact size of the lancet device 600' .

Furthermore, as for the needle cap 170' of the lancet device 600' , it has a pair of wings 175' extending symmetrically and backwardly (see Figs. 19 and 20). As shown in Figs. 19 and 20, the tip 175' of one of the pair of the wings 175' is located beneath the front end of the pushing portion 410' of the trigger part, which can prevent the pushing portion 410' from being pushed into the device at the time before the pricking operation. In contrast, after the needle cap 170' is removed, the wing 175 is also removed, so that the tip 175a' of the wing 175' is no longer located beneath the front end of the pushing portion 410' . As a result, the pushing portion 410' of the trigger part can be pushed into the device. Therefore, the removal of the needle cap 170' from the pricking needle makes it possible to push the pushing portion 410' of the trigger part into the device so as to launch the pricking needle.

### (Lancet Device of Embodiment Shown in Figs. 29 to 39)

A lancet device 600" according to the embodiment of Figs. 29 to 39 has substantially the same functions as those of the above lance device 600, but mainly differs from the above lancet device 600 in form of the trigger part (particularly, a rotation starting mechanism of the driving shaft component, and a releasing mechanism of the movement inhibition of the needle shaft component), arrangement of the compressed spring, and the like.

As shown in Fig. 31, the lancet device 600" is mainly composed of a lancet pricking part 100", a pressing part 200", a housing 300" and a case-shaped trigger part 400". As can be seen from the embodiment shown in Figs. 29 to 31, the pricking part 100" is accommodated in the case-shaped trigger part 400" of the lancet device 600". The case-shaped trigger part 400" accommodating therein the pricking part 100" is surrounded by the housing 300" such that only the tip portion of the trigger part is exposed. In other words, the case-shaped trigger part 400" is entirely positioned within the housing 300" such that a pricking opening 430" provided at the tip portion of the case-shaped trigger part 400" protrudes outwardly from a front opening end 303" of the housing 300". The term "case-shaped trigger part" as used herein substantially means that a part for launching the pricking needle has a sheath or cylindrical shape. Thus, the trigger part of the lancet device 600" takes such a form as to accommodate therein the pricking part as shown in Fig. 31.

As can be seen from the embodiments shown in Figs 29 to 31, the needle cap 170" has a front-sided portion 175" which protrudes outwardly from the pricking opening 430" of the case-shaped trigger part. The pair of rearward extending parts 175a" which extend backwardly from the sides of the front-sided portion 175" is adjacent to a front opening end 303" of the housing, which can prevent the case-shaped trigger part 400" from being pushed into the housing 300".

After the needle cap 170" is removed from the lancet pricking part 100" for the purpose of carrying out the pricking operation, the case-shaped trigger part 400" can be pushed backwardly. Specifically, when the external force is applied to the pricking opening 430" provided at the tip portion of the case-shaped trigger 400" with no needle cap 170" disposed, the case-shaped trigger part 400" can move backwardly with respect to the housing 300", which leads to an achievement of the pushing of the case-shaped trigger part 400" into the housing.

As shown in Figs. 30, 31, and 36, the case-shaped trigger part 400" has a first flexible portion 470" on its side. The first flexible portion 470" is in a form of a small plate with its fixed end 471" and its free end 472", and thus can be bent outwardly by the external force (particularly see Fig. 36). At a point in time before the pricking operation, as shown in Fig. 38(a), the first flexible portion 470" is in abutment against the driving shaft component 140" of the pricking part, whereby the pressing part 200" coupled to the driving shaft component 140" is kept compressed. When the tip of the case-shaped trigger part (that is, the pricking opening 430" of the case-shaped trigger part) is pushed toward the inside of the housing, the first flexible portion 470" of the case-shaped trigger part slides on a first continuous projecting portion (330" ) provided on the inner wall of the housing (see Fig. 39). As a result, the first flexible portion 470" expands outwardly. The outward expansion of the first flexible portion 470" makes it possible to cease the contact between the first flexible portion 470" and the driving shaft component 140" (see Figs. 38(a) to 38(c)), and thereby allowing the compressed spring 200" to extend to press the driving shaft component 140" in the rotation direction. This results in a movement of the needle shaft component 120" both in the pricking direction and in the opposite direction thereto (see Figs. 38(c) and 38(d)). The first continuous projecting portion (330") provided on the inner wall of the housing preferably has a slope surface 330a" on its side so that the first flexible portion 470" appropriately expands outwardly (see Fig. 32). The free end 472" of the first flexible portion 470" is preferably provided with a sloped portion 472a" which is capable of making contact with the slope surface 330a" of the first continuous projecting portion (see Fig. 36).

As shown in Figs. 30, 31, and 36, it is preferred that the case-shaped trigger part 400" further has a second flexible portion 490" in addition to the first flexible portion 470". Similarly to the first flexible portion 470", the second flexible portion 490" is also in a form of a small plate with its fixed end 491" and its free end 492", and thus can be bent outwardly by the external force (particularly see Fig. 36). As shown in Fig. 38(a), at a point in time before the pricking operation, the second flexible portion 490" is in engagement with the needle shaft component 120" , which can prevent the displacement of the needle shaft component 120" during the removal of the needle cap 170" from the tip of the pricking needle. When the tip portion of the case-shaped trigger part (that is, the pricking opening 430" of the case-shaped trigger part) is pushed toward the inside of the housing at a point in time after the removal of the needle cap 170", the second flexible portion 490" slides on the second continuous projecting portion (360") provided on the inner wall of the housing (see Fig. 39). As a result, the second flexible portion 490" expands outwardly. The outward expansion of the second flexible portion 490" makes it possible to cease the engagement between the second flexible portion 490" and the needle shaft component 120" (see Figs. 38(a) to 38(c)). That is, at a point in time before the case-shaped trigger part 400" is pushed, the second flexible portion 490" is in engagement with a recess portion 127" of the needle shaft component 120' (see Fig. 35 as to the recess portion 127" of the needle shaft component), whereby the needle shaft component 120" can be prevented from being displaced and moved during the removal of the needle cap 170'. While on the other hand, when the case-shaped trigger part 400" is pushed into the device at a point in time after the removal of the needle cap 170", the second flexible portion 490" can slide on the second continuous projecting portion (360") provided on the inner wall of the housing to expand outwardly, which makes it possible to cease the engagement between the second flexible portion 490" and the needle shaft component 120". After the cease of such engagement, nothing prevents the movement of the needle shaft component 120' , which enables the launching of the needle shaft component 120" , that is, the launching of the pricking needle. In this regard, it is preferred that the cease of the engagement between the second flexible portion 490" and the needle shaft component 120" is performed prior to or substantially at the same time as the cease of the contact between the first flexible component 470" and the driving shaft component 140" . The second continuous projecting portion (360") provided on the inner wall of the housing preferably has a slope surface 360a" on its side so that the second flexible component 490" appropriately expands outwardly (see Fig. 32). It is preferred that the free end 492" of the second flexible portion 490" is provided with a sloped portion 492a" which is capable of making contact with the slope surface 360a" of the second continuous projecting portion (see Fig. 36).

Since the lancet pricking part 100" is accommodated within the case-shaped trigger part 400" according to the embodiment of the lancet device 600", the case-shaped trigger part 400" preferably has a guide rail 450" for a sliding movement of the needle shaft component 120" (see Fig. 36). That is, when the needle shaft component 120" moves in the pricking direction and in the opposite direction thereto during the pricking operation, the needle shaft component 120" preferably slides on the guide rail 450" of the case-shaped trigger part 400". The guide rail 450" of the lancet device 600" may have the form similar to that of the above lancet device 600. As shown in Fig. 36, the guide rail 450" is preferably composed of two opposed elongate portions (450a", 450b") which protrude vertically with respect to the inner wall of the case-shaped trigger part 400". Such guide rail 450" can ensure the sliding movement of the needle shaft component 120". Specifically, when the needle shaft component 120" of the lancet pricking part 100" moves in the pricking direction and the direction opposed thereto, the needle shaft component 120" slides while being in contact with the guide rail 450" of the case-shaped trigger part 400", which leads to an achievement of the linearity of the needle (see Figs. 38(c) and 38(d)).

The arrangement of the compressed spring 200" in the lancet device 600" is the same as that of the above lancet device 600' . That is, a part or most of the compressed spring 200" which is coupled to the driving shaft component 140" is accommodated within a recessed portion of the driving shaft component 140" (see Fig. 38(a)). As shown in Figs. 34 and 35, the recessed portion 147" of the driving shaft component 140" preferably has a complementary shape with respect to the entire shape of the compressed spring 200". In this embodiment shown in Figs. 29 to 39, the entire size of the device can be reduced (especially, the longitudinal direction dimension) by the disposed length of the compressed spring 200" in the driving shaft component 140", which leads to a more compact size of the lancet device 600".

In the lancet device 600" according to the embodiment of Figs. 29 to 39, the pricking operation can be performed by pressing the lancet device against the "predetermined region to be pricked" with a grip component 380" (which is provided at the outer surface of the housing (see Fig. 29)) gripped with the fingers. The pressing of the lancet device against the "predetermined region to be pricked" causes the tip portion of the case-shaped trigger part to be pushed into the housing. That is, according to the lancet device 600" of Figs. 29 to 39, the pricking operation can be performed by a "stamping" operation as shown in Fig. 40. This operation differs from those of the lancet devices 600 and 600' (see Fig. 41). In this way, the lancet devices can suitably change and modify the pricking operation according to actual applications and needs.

Although a few embodiments of the present invention have been hereinbefore described, such embodiments are only for illustrative purpose regarding the typical examples, and thus the present invention is not limited to these embodiments. It will be readily appreciated by those skilled in the art that various modifications are possible without departing from the scope of the invention. For example, the following modified embodiments are possible.

In the accompanying drawings, the pricking needle 120a has a "needle form" whose uppermost is wholly sharpened, but is not necessarily limited thereto. For example, the pricking needle 120a may have a "blade form" having one side face of its tip sharpened.

### EXAMPLES

In order to confirm the effects of the improved linearity of the needle and the improved prevention of the needle wobble in the lancet device, the following tests were performed.

The device shown in Fig. 5 was used as the lancet device of the invention (Example 1), whereas a "plunger lancet device equipped with a stick-like plunger" was used as a prior art device of Comparative Example (Comparative Example 1: Prior art device "A", Comparative Example 2: Prior art device "B").

The results are shown in the following Table 1 as well as Fig. 42.

**[Table 1]**

| Item | Protruding length of needle | | | | Wobbling of tip of needle | | | | | | | | |
|---|---|---|---|---|---|---|---|---|---|---|---|---|---|
| Device | Device of Present Invention (Example 1) | | Prior art device "A" (Comparative Example 1) | Prior art device "B" (Comparative Example 2) | Device of Present Invention (Example 1) | | | | Prior art device "A" (Comparative Example 1) | | | Prior art device "B" (Comparative Example 2) | |
| | Setting for protruding length of needle: 1 mm | Setting for protruding length of needle: 1.5 mm | Setting for protruding length of needle: 1.5 mm | Setting for protruding length of needle: 1.8 mm | Setting for protruding length of needle: 1 mm | | Setting for protruding length of needle: 1.5 mm | | Setting for protruding length of needle: 1.5 mm | | | Setting for protruding length of needle: 1.8 mm | |
| | | | | | Front side | Lateral side | Front side | Lateral side | Front side | Lateral side | | Front side | Lateral side |
| Measurement equipment | High-speed camera | | | | High-speed camera | | | | | | | | |
| Unit | mm | | | | mm | | | | | | | | |
| 1 | 1.12 | 1.61 | 1.52 | 1.91 | 0.04 | 0.03 | 0.06 | 0.03 | 0.35 | | 0.44 | | |
| 2 | 1.04 | 1.60 | 1.58 | 1.82 | 0.04 | 0.02 | 0.07 | 0.04 | 0.13 | | 0.42 | | |
| 3 | 1.08 | 1.54 | 1.89 | 1.74 | 0.03 | 0.03 | | 0.02 | 0.14 | | 0.37 | | |
| 4 | 1.12 | 1.58 | | | | | | | | | | | |
| 5 | 1.08 | 1.57 | | | | | | | | | | | |
| 6 | 1.06 | | | | | | | | | | | | |
| AVE | 1.083 | 1.580 | 1.663 | 1.823 | 0.037 | 0.027 | 0.065 | 0.030 | 0.207 | - | 0.410 | | - |
| MAX | 1.12 | 1.61 | 1.89 | 1.91 | 0.04 | 0.03 | 0.07 | 0.04 | 0.35 | - | 0.44 | | - |
| MIN | 1.04 | 1.54 | 1.52 | 1.74 | 0.03 | 0.02 | 0.06 | 0.02 | 0.13 | - | 0.37 | | - |
| R | 0.08 | 0.07 | 0.37 | 0.17 | 0.01 | 0.01 | 0.01 | 0.02 | 0.22 | - | 0.07 | | - |
| STD | 0.032 | 0.027 | 0.199 | 0.085 | 0.006 | 0.006 | 0.007 | 0.010 | 0.124 | - | 0.036 | | - |

As can be seen from the results shown in Table 1 and Fig. 42, the lancet device according to embodiments of the present invention has a very high level of the linear track of the pricking needle, and also effectively prevents the wobble of the tip of the needle.

### INDUSTRIAL APPLICABILITY

The lancet pricking device has preventing functions of "twice pricking phenomenon" and "re-use", and also has an improved pricking path of the pricking needle. Accordingly, the lance pricking device can be not only used to take blood from a patient with diabetes, but also suitably used for various other applications where the blood sampling is needed.

### REFERENCE NUMERALS

100 Pricking part (Lancet pricking part)
120 Needle shaft component
120a Pricking needle
120a1 Tip of Pricking needle
125 Elastic portion of needle shaft component
127 Recess portion of needle shaft component for engaging with movement-inhibiting component
133 Rear end of pricking part
140 Driving shaft component
145 Cushion portion of driving shaft component
160 Intermediate shaft component
180(180a,180b) Joint component
170 Needle cap
172 Holding portion of needle cap
200 Pressing part (e.g., spring part)
300 Housing
310 Pricking opening
320 Housing's contact surface for pressing part
350(350a,350b) Guide rail
350a1 Tapered portion
370 Boss of housing
390 Trigger opening of housing
400 Trigger part
410 Pushing portion of trigger part
410a Front-sided upper portion of trigger part
440 Backwardly-extending elongate component of trigger part
460 Movement-inhibiting component of trigger part
460a Raised portion of movement-inhibiting component
600 Lancet device

100' Pricking part (Lancet pricking part)
120' Needle shaft component
120a' Pricking needle
120a1' Tip of Pricking needle
125' Elastic portion of needle shaft component
127' Recess portion of needle shaft component for engaging with movement-inhibiting component (460')
140' Driving shaft component
145' Cushion portion of driving shaft component
160' Intermediate shaft component
180' (180a', 180b') Joint component
170' Needle cap
172' Holding portion of needle cap
200' Pressing part (e.g., spring part)
300' Housing
310' Pricking opening
320a' Housing's contact surface for pressing part
350' (350a', 350b') Guide rail
370' Boss of housing
390' Trigger opening of housing
400' Trigger part
410' Pushing portion of trigger part
440' Backwardly-extending elongate component of trigger part
460' Movement-inhibiting component of trigger part
465' Node of movement-inhibiting component
468' "Free end equipped with protrusion" of movement-inhibiting component
600' Lancet device

100" Pricking part (Lancet pricking part)
120" Needle shaft component
120a" Pricking needle
120a1" Tip of Pricking needle
125" Elastic portion of needle shaft component
140" Driving shaft component
145" Cushion portion of driving shaft component
160" Intermediate shaft component
180" (180a", 180b") Joint component
170" Needle cap
172" Holding portion of needle cap
175" Front-sided portion of needle cap
175a" Pair components extending backwardly from the side of front-sided portion 175"
200" Pressing part (e.g., spring part)
300" Housing
330" First continuous projecting portion
330a" Slope surface provided in side face of first continuous projecting portion
360" Slope surface provided in side face of second continuous projecting portion
400" Case-shaped trigger part
420" Contact surface for pressing part, provided in case-shaped trigger part
430" Front end portion (pricking opening) of case-shaped trigger part
450" (450a', 450b') Guide rail of case-shaped trigger part
470" First flexible portion
471" Free end of first flexible portion
472" Fixed end of first flexible portion
472a" Sloped portion of first flexible portion
490" Second flexible portion
491" Free end of second flexible portion
492" Fixed end of second flexible portion
492a" Sloped portion of second flexible portion
600" Lancet device

## Claims

1. A lancet device comprising a pricking part and a pressing part,
wherein the pricking part (100) comprises a needle shaft component (120) equipped with a pricking needle (120a), a driving shaft component (140), and an intermediate shaft component (160) therebetween, the needle shaft component (120), the driving shaft component (140) and the intermediate shaft component (160) being connected to each other via a joint component (180),
wherein the driving shaft component (140) of the pricking part (100) is at least partly rotated by a pressing action of the pressing part (200), and thereby the intermediate shaft component (160) of the pricking part (100) is displaced causing the needle shaft component (120) to move in a pricking direction, **characterized in that** the needle shaft component (120), the driving shaft component (140), the intermediate shaft component (160) and the joint component (180) therebetween are all in an integrally formed shape with each other in the pricking part (100), wherein, when the needle shaft component moves in the pricking direction, an elastic portion(125) having a hollow structure of the needle shaft component (120) is brought into contact with an inner wall of a lancet housing so as to receive a force from a surface of a guide (350) on the inner wall of the housing.

2. The lancet device according to claim 1, wherein a displacement direction of the intermediate shaft component (160) and the pricking direction of the needle shaft component (120) are not in the same line as each other.

3. The lancet device according to claim 1 or claim 2, wherein, during the pressing action of the pressing part (200), the needle shaft component (120) of the pricking part (100) moves in the pricking direction and subsequently the needle shaft component (120) moves backwardly in the opposite direction to the pricking direction.

4. The lancet device according to any one of claims 1 to 3, wherein the intermediate shaft component (160) of the pricking part is displaced such that the intermediate shaft component (160) moves in a direction perpendicular to the pricking direction of the needle shaft component (120).

5. The lancet device according to claim 4, wherein a rear end of the intermediate shaft component (160) moves in the direction perpendicular to the pricking direction of the needle shaft component (120) such that a front end of the intermediate shaft component (160) serves as an axis of the movement of the rear end.

6. The lancet device according to any one of claims 2 to 5, wherein the joint component (180) is in a thin form in the pricking part (100).

7. The lancet device according to any one of claims 1 to 6, wherein the joint component (180) is flexible.

8. The lancet device according to any one of claims 4 to 7 when appendant to claim 3, wherein the pressing part (200) is a spring part, and
wherein, during the spring part in a compressed state is being released to extend, the driving shaft component (140) is pressed in a rotation direction thereof by the spring part, and thereby the intermediate shaft component (160) of the pricking part (100) is displaced causing the needle shaft of the pricking part (100) to move both in the pricking direction and an opposite direction thereto.

9. The lancet device according to any one of claims 4 to 8 when appendant to claim 3, wherein the housing (300) accommodates the pricking part and the pressing part therein.
wherein the guide on the inner wall of the housing is a guide rail for a slide movement of the needle shaft component, and
wherein, when the needle shaft component moves in the pricking direction and the opposite direction thereto, the needle shaft component slides on the guide rail.

10. The lancet device according to claim 9, wherein the elastic portion (125) has a hollow structure, and
wherein, when the needle shaft component moves in the pricking direction and the opposite direction thereto, the elastic portion of the needle shaft component slides on the guide rail.

11. The lancet device according to claim 9 or 10, further comprising a trigger part (400),
wherein a rear end of the trigger part (400) is in abutment with the driving shaft component (140) of the pricking part such that the spring part coupled to the driving shaft component (140) is held in its compressed state, and
wherein, a pushing of a front portion of the trigger part (400) toward an inside of the housing (300) causes a ceasing of the abutment of the rear end of the trigger part (400) with the driving shaft component (140) of the pricking part, and thereby the driving shaft component (140) is pressed in the rotation direction thereof by the spring part.

12. The lancet device according to claim 11, wherein the pricking part is equipped with a needle cap (170) for protecting the tip of the pricking needle (120a), and the trigger part is equipped with a movement-inhibiting component for inhibiting a movement of the pricking part (100),
wherein, at a point in time before a pricking operation, the movement-inhibiting component of the trigger part (400) and the needle shaft component (120) of the pricking part are capable of making abutment with each other, and thereby a displacement of the needle shaft component (120) is prevented during a removal of the needle cap (170) from the tip of the pricking needle.

13. The lancet device according to claim 8, further comprising a case-shaped trigger part (400) and a housing (300),
wherein the pricking part (100) is accommodated within the case-shaped trigger part,
wherein the case-shaped trigger part in which the pricking part (100) is accommodated is surrounded by the housing (300) such that only a tip portion of the trigger part (400) is exposed from the housing,
wherein the case-shaped trigger part comprises a first flexible portion which is in abutment with the driving shaft component (140) of the pricking part at a point in time before a pricking operation, and thereby the spring part coupled to the driving shaft component (140) is held in its compressed state, and
wherein, a pushing of the tip portion of the case-shaped trigger part (400) toward an inside of the housing (300) for the pricking operation forces the first flexible portion of the case-shaped trigger part to slide on a first continuous projecting portion provided on an inner wall of the housing, and consequently the first flexible portion is displaced outwardly to cease the abutment of the first flexible portion with the driving shaft component (140), and thereby the driving shaft component (140) is pressed in the rotation direction thereof by the spring part.

14. The lancet device according to claim 13, wherein the case-shaped trigger part (400) further comprises a second flexible portion in addition to the first flexible portion, wherein the second flexible portion is in engagement with the needle shaft component (120) at a point in time before the pricking operation, and thereby a displacement of the needle shaft component (120) is prevented during a removal of the needle cap (170) from the tip of the pricking needle, and
wherein a pushing of the tip portion of the case-shaped trigger part (400) toward the inside of the housing (300) for the pricking operation forces the second flexible portion of the case-shaped trigger part (400) to slide on a second continuous projecting portion provided on an inner wall of the housing, and consequently the second flexible portion is displaced outwardly to cease the abutment of the second flexible portion with the needle shaft component (120).

## Patentansprüche

1. Lanzettenvorrichtung mit einem Stechteil und einem Drückteil,
wobei das Stechteil (100) eine mit einer Stechnadel (120a) ausgestattete Nadelschaftkomponente (120), eine Antriebsschaftkomponente (140) und eine Zwischenschaftkomponente (160) dazwischen aufweist, wobei die Nadelschaftkomponente (120), die Antriebsschaftkomponente (140) und die Zwischenschaftkomponente (160) miteinander über eine Gelenkkomponente (180) verbunden sind,
wobei die Antriebsschaftkomponente (140) des Stechteils (100) durch einen Drückvorgang des Drückteils (200) zumindest teilweise gedreht wird, und dadurch die Zwischenschaftkomponente (160) des Stechteils (100) verschoben wird, wobei bewirkt wird, dass sich die Nadelschaftkomponente (120) in eine Stichrichtung bewegt, **dadurch gekennzeichnet, dass** die Nadelschaftkomponente (120), die Antriebsschaftkomponente (140), die Zwischenschaftkomponente (160) und die Gelenkkomponente (180) dazwischen alle in einer integriert ausgebildeten Form miteinander in dem Stechteil (100) sind, wobei, wenn sich die Nadelschaftkomponente in die Stichrichtung bewegt, ein elastischer Abschnitt (125), der eine hohle Struktur der Nadelschaftkomponente (120) aufweist, mit einer Innenwand eines Lanzettengehäuses in Kontakt gebracht wird, um eine Kraft von einer Oberfläche einer Führung (350) an der Innenwand des Gehäuses zu empfangen.

2. Lanzettenvorrichtung nach Anspruch 1, wobei eine Verschiebungsrichtung der Zwischenschaftkomponente (160) und die Stichrichtung der Nadelschaftkomponente (120) nicht in der gleichen Linie miteinander sind.

3. Lanzettenvorrichtung nach Anspruch 1 oder Anspruch 2, wobei, während des Drückvorgangs des Drückteils (200), sich die Nadelschaftkomponente (120) des Stechteils (100) in die Stichrichtung bewegt und sich nachfolgend die
Nadelschaftkomponente (120) rückwärts in die entgegengesetzte Richtung zu der Stichrichtung bewegt.

4. Lanzettenvorrichtung nach einem der Ansprüche 1 bis 3, wobei die Zwischenschaftkomponente (160) des Stechteils derart verschoben wird, dass sich die Zwischenschaftkomponente (160) in eine Richtung senkrecht zu der Stichrichtung der Nadelschaftkomponente (120) bewegt.

5. Lanzettenvorrichtung nach Anspruch 4, wobei sich ein hinteres Ende der Zwischenschaftkomponente (160) derart in die Richtung senkrecht zu der Stichrichtung der Nadelschaftkomponente (120) bewegt, dass ein vorderes Ende der Zwischenschaftkomponente (160) als eine Achse der Bewegung des hinteren Endes dient.

6. Lanzettenvorrichtung nach einem der Ansprüche 2 bis 5, wobei die Gelenkkomponente (180) in dem Stechteil (100) in einer dünnen Form ist.

7. Lanzettenvorrichtung nach einem der Ansprüche 1 bis 6, wobei die Gelenkkomponente (180) flexibel ist.

8. Lanzettenvorrichtung nach einem der Ansprüche 4 bis 7, bei Abhängigkeit von Anspruch 3, wobei das Drückteil (200) ein Federteil ist, und
wobei, während das Federteil in einem zusammengedrückten Zustand gelöst wird, um sich zu erstrecken, die Antriebsschaftkomponente (140) durch das Federteil in eine Rotationsrichtung von ihr gedrückt wird, und wobei dadurch die Zwischenschaftkomponente (160) des Stechteils (100) verschoben wird, wobei verursacht wird, dass sich der Nadelschaft des Stechteils (100) in beide, die Stichrichtung und eine entgegengesetzte Richtung dazu bewegt.

9. Lanzettenvorrichtung nach einem der Ansprüche 4 bis 8, bei Abhängigkeit von Anspruch 3, wobei das Gehäuse (300) das Stechteil und das Drückteil darin aufnimmt,
wobei die Führung an der Innenwand des Gehäuses eine Führungsschiene für eine Gleitbewegung der Nadelschaftkomponente ist, und
wobei, wenn sich die Nadelschaftkomponente in die Stichrichtung und die entgegengesetzte Richtung dazu bewegt, die Nadelschaftkomponente auf der Führungsschiene gleitet.

10. Lanzettenvorrichtung nach Anspruch 9, wobei der elastische Abschnitt (125) eine hohle Struktur aufweist, und
wobei, wenn sich die Nadelschaftkomponente in die Stichrichtung und die entgegengesetzte Richtung dazu bewegt, der elastische Abschnitt der Nadelschaftkomponente auf der Führungsschiene gleitet.

11. Lanzettenvorrichtung nach Anspruch 9 oder 10, ferner mit einem Auslöseteil (400),
wobei ein hinteres Ende des Auslöseteils (400) derart in Angrenzung mit der Antriebsschaftkomponente (140) des Stechteils ist, dass das an die Antriebsschaftkomponente (140) gekoppelte Federteil in seinem zusammengedrückten Zustand gehalten wird, und
wobei ein Schieben eines vorderen Abschnitts des Auslöseteils (400) zu einer Innenseite des Gehäuses (300) hin ein Beenden der Angrenzung des hinteren Endes des Auslöseteils (400) mit der Antriebsschaftkomponente (140) des Stechteils bewirkt, und dadurch die Antriebsschaftkomponente (140) durch das Federteil in die Rotationsrichtung von ihr gedrückt wird.

12. Lanzettenvorrichtung nach Anspruch 11, wobei das Stechteil mit einer Nadelkappe (170) zum Schützen der Spitze der Stechnadel (120a) ausgestattet ist, und das Auslöseteil mit einer Bewegungsblockierkomponente zum Hemmen einer Bewegung des Stechteils (100) ausgestattet ist,
wobei, zu einem Zeitpunkt vor einem Stichvorgang, die Bewegungsblockierkomponente des Auslöseteils (400) und die Nadelschaftkomponente (120) des Stechteils imstande zum Ausführen einer Angrenzung miteinander sind, und dadurch eine Verschiebung der Nadelschaftkomponente (120) während eines Entfernens der Nadelkappe (170) von der Spitze der Stechnadel verhindert wird.

13. Lanzettenvorrichtung nach Anspruch 8, ferner mit einem kastenförmigen Auslöseteil (400) und einem Gehäuse (300),
wobei das Stechteil (100) innerhalb des kastenförmigen Auslöseteils aufgenommen ist,
wobei das kastenförmige Auslöseteil, in welchem das Stechteil (100) aufgenommen ist, durch das Gehäuse (300) derart umgeben ist, dass lediglich ein Spitzenabschnitt des Auslöseteils (400) von dem Gehäuse freiliegt,
wobei das kastenförmige Auslöseteil einen ersten flexiblen Abschnitt umfasst, welcher in Angrenzung mit der Antriebsschaftkomponente (140) des Stechteils zu einem Zeitpunkt vor einem Stichvorgang ist, und dadurch das an die Antriebsschaftkomponente (140) gekoppelte Federteil in seinem zusammengedrückten Zustand gehalten wird, und
wobei ein Schieben des Spitzenabschnitts des kastenförmigen Auslöseteils (400) zu einer Innenseite des Gehäuses (300) hin für den Stichvorgang den ersten flexiblen Abschnitt des kastenförmigen Auslöseteils zwingt, auf einem ersten kontinuierlichen Vorsprungsabschnitt zu gleiten, der an einer Innenwand des Gehäuses vorgesehen ist, und somit der erste flexible Abschnitt nach außen verschoben wird, um die Angrenzung des ersten flexiblen Abschnitts mit der Antriebsschaftkomponente (140) zu beenden, und dadurch die Antriebsschaftkomponente (140) durch das Federteil in die Rotationsrichtung von ihr gedrückt wird.

14. Lanzettenvorrichtung nach Anspruch 13, wobei das kastenförmige Auslöseteil (400) ferner einen zweiten flexiblen Abschnitt zusätzlich zu dem ersten flexiblen Abschnitt aufweist,
wobei der zweite flexible Abschnitt mit der Nadelschaftkomponente (120) zu einem Zeitpunkt vor dem Stichvorgang im Eingriff ist, und dadurch eine Verschiebung der Nadelschaftkomponente (120) während eines Entfernens der Nadelkappe (170) von der Spitze der Stechnadel verhindert wird, und
wobei ein Schieben des Spitzenabschnitts des kastenförmigen Auslöseteils (400) zu der Innenseite des Gehäuses (300) hin für den Stichvorgang den zweiten flexiblen Abschnitt des kastenförmigen Auslöseteils (400) zwingt, auf einem zweiten kontinuierlichen Vorsprungsabschnitt zu gleiten, der an einer Innenwand des Gehäuses vorgesehen ist, und somit der zweite flexible Abschnitt nach außen verschoben wird, um die Angrenzung des zweiten flexiblen Abschnitts mit der Nadelschaftkomponente (140) zu beenden.

## Revendications

1. Dispositif de lancette comprenant une partie de piqûre et une partie de pression,
dans lequel la partie de piqûre (100) comprend un composant d'élément d'aiguille (120) équipé d'une aiguille de piqûre (120a), un composant d'élément d'entraînement (140), et un composant d'élément intermédiaire (160) entre eux, le composant d'élément d'aiguille (120), le composant d'élément d'entraînement (140) et le composant d'élément intermédiaire (160) étant connectés ensemble via un composant de joint (180),
dans lequel le composant d'élément d'entraînement (140) de la partie de piqûre (100) est au moins en partie mis en rotation par une action de pression de la partie de pression (200), et ainsi le composant d'élément intermédiaire (160) de la partie de piqûre (100) est déplacé faisant se déplacer le composant d'élément d'aiguille (120) dans une direction de piqûre, **caractérisé en ce que** le composant d'élément d'aiguille (120), le composant d'élément d'entraînement (140), le composant d'élément intermédiaire (160) et le composant de joint (180) entre eux sont tous formés d'un seul bloc les uns avec les autres dans la partie de piqûre (100), dans lequel quand le composant d'élément d'aiguille se déplace dans la direction de piqûre, une partie élastique (125) ayant une structure creuse du composant d'élément d'aiguille (120) est amenée en contact avec une paroi intérieure d'un logement de lancette de façon à recevoir une force depuis une surface d'un guide (350) sur la paroi intérieure du logement.

2. Dispositif de lancette selon la revendication 1, dans lequel une direction de déplacement du composant d'élément intermédiaire (160) et la direction de piqûre du composant d'élément d'aiguille (120) ne sont pas alignées.

3. Dispositif de lancette selon la revendication 1 ou la revendication 2, dans lequel, pendant l'action de pression de la partie de pression (200), le composant d'élément d'aiguille (120) de la partie de piqûre (100) se déplace dans la direction de piqûre et ensuite le composant d'élément d'aiguille (120) se déplace vers l'arrière dans la direction opposée à la direction de piqûre.

4. Dispositif de lancette selon l'une quelconque des revendications 1 à 3, dans lequel le composant d'élément intermédiaire (160) de la partie de piqûre est déplacé de telle manière que le composant d'élément intermédiaire (160) se déplace dans une direction perpendiculaire à la direction de piqûre du composant d'élément d'aiguille (120).

5. Dispositif de lancette selon la revendication 4, dans lequel une extrémité arrière du composant d'élément intermédiaire (160) se déplace dans la direction perpendiculaire à la direction de piqûre du composant d'élément d'aiguille (120) de telle manière qu'une extrémité avant du composant d'élément intermédiaire (160) sert comme un axe du mouvement de l'extrémité arrière.

6. Dispositif de lancette selon l'une quelconque des revendications 2 à 5, dans lequel le composant de joint (180) est sous une forme fine dans la partie de piqûre (100).

7. Dispositif de lancette selon l'une quelconque des revendications 1 à 6, dans lequel le composant de joint (180) est flexible.

8. Dispositif de lancette selon l'une quelconque des revendications 4 à 7 dépendant de la revendication 3, dans lequel la partie de pression (200) est une partie de ressort, et
dans lequel, pendant que la partie de ressort dans un état comprimé est relâchée pour s'allonger, le composant d'élément d'entraînement (140) est pressé dans une direction de rotation de celui-ci par la partie de ressort, et donc le composant d'élément intermédiaire (160) de la partie de piqûre (100) est déplacé faisant se déplacer l'axe d'aiguille de la partie de piqûre (100) à la fois dans la direction de piqûre et dans la direction qui lui est opposée.

9. Dispositif de lancette selon l'une quelconque des revendications 4 à 8 dépendant de la revendication 3, dans lequel le logement (300) loge en lui la partie de piqûre et la partie de pression,
dans lequel le guide sur la paroi latérale du logement est un rail de guidage pour un mouvement coulissant du composant d'élément d'aiguille, et
dans lequel, quand le composant d'élément d'aiguille se déplace dans la direction de piqûre et dans la direction qui lui est opposée, le composant d'élément d'aiguille coulisse sur le rail de guidage.

10. Dispositif de lancette selon la revendication 9, dans lequel la partie élastique (125) a une structure creuse, et
dans lequel, quand le composant d'élément d'aiguille se déplace dans la direction de piqûre et dans la direction qui lui est opposée, la partie élastique du composant d'élément d'aiguille coulisse sur le rail de guidage.

11. Dispositif de lancette selon la revendication 9 ou 10, comprenant en outre une partie de déclenchement (400),
dans lequel une extrémité arrière de la partie de déclenchement (400) est en butée avec le composant d'élément d'entraînement (140) de la partie de piqûre de telle manière que la partie de ressort couplée au composant d'élément d'entraînement (140) est maintenue dans son état comprimé, et
dans lequel, une poussée d'une partie avant de la partie de déclenchement (400) vers un intérieur du logement (300) provoque la fin de la butée de l'extrémité arrière de la partie de déclenchement (400) avec le composant d'élément d'entraînement (140) de la partie de piqûre, et ainsi le composant d'élément d'entraînement (140) est pressé dans la direction de rotation de celui-ci par la partie de ressort.

12. Dispositif de lancette selon la revendication 11, dans lequel la partie de piqûre est équipée d'un capuchon d'aiguille (170) pour protéger la pointe de l'aiguille de piqûre (120a), et la partie de déclenchement est équipée d'un composant de blocage de mouvement pour empêcher un mouvement de la partie de piqûre (100),
dans lequel, à un moment avant l'opération de piqûre, le composant de blocage de mouvement de la partie de déclenchement (400) et le composant d'élément d'aiguille (120) de la partie de piqûre peuvent venir en butée l'un avec l'autre, et ainsi un déplacement du composant d'élément d'aiguille (120) est empêché pendant un enlèvement du capuchon d'aiguille (170) de la pointe de l'aiguille de piqûre.

13. Dispositif de lancette selon la revendication 8, comprenant en outre une partie de déclenchement en forme de boîtier (400) et un logement (300),
dans lequel la partie de piqûre (100) est logée dans la partie de déclenchement en forme de boîtier,
dans lequel la partie de déclenchement en forme de boîtier dans laquelle la partie de piqûre (100) est logée est entourée par le logement (300) de telle manière que seule une partie de pointe de la partie de déclenchement (400) est exposée depuis le logement,
dans lequel la partie de déclenchement en forme de boîtier comprend une première partie flexible qui est en butée avec le composant d'élément d'entraînement (140) de la partie de piqûre en un point de temps avant une opération de piqûre, et ainsi la partie de ressort couplée au composant d'élément d'entraînement (140) est maintenue dans son état comprimé, et
dans lequel, une poussée de la partie de pointe de la partie de déclenchement en forme de boîtier (400) vers un intérieur du logement (300) pour l'opération de piqûre force la première partie flexible de la partie de déclenchement en forme de boîtier à coulisser sur une première partie de saillie continue placée sur une paroi intérieure du logement, et par conséquent la première partie flexible est déplacée vers l'extérieur pour faire cesser la butée de la première partie flexible avec le composant d'élément d'entraînement (140), et ainsi le composant d'élément d'entraînement (140) est pressé dans la direction de rotation de celui-ci par la partie de ressort.

14. Dispositif de lancette selon la revendication 13, dans lequel la partie de déclenchement en forme de boîtier (400) comprend en outre une seconde partie flexible en plus de la première partie flexible, dans lequel la seconde partie flexible est en prise avec le composant d'élément d'aiguille (120) à un moment avant l'opération de piqûre, et ainsi un déplacement du composant d'élément d'aiguille (120) est empêché pendant un enlèvement du capuchon d'aiguille (170) de la pointe de l'aiguille de piqûre, et
dans lequel une poussée de la partie de pointe de la partie de déclenchement en forme de boîtier (400) vers l'intérieur du logement (300) pour l'opération de piqûre force la seconde partie flexible de la partie de déclenchement en forme de boîtier (400) à coulisser sur une seconde partie saillante continue placée sur une paroi intérieure du logement, et par conséquent la seconde partie flexible est déplacée vers l'extérieur pour faire cesser la butée de la seconde partie flexible avec le composant d'élément d'aiguille (120).
